# EUROPEAN PATENT SPECIFICATION

(11) **EP 0 542 887 B1**
(45) Date of publication and mention of the grant of the patent: **02.12.1998**
(21) Application number: 91915657.0
(22) Date of filing: 09.08.1991
(51) Int. Cl.: C12Q 1/68, C12N 15/00, A01N 43/04, A61K 31/70, C07H 15/12, C07H 17/00

(54) **PSORALEN CONJUGATED METHYLPHOSPHONATE OLIGONUCLEOTIDES AS THERAPEUTIC AGENTS FOR CHRONIC MYELOGENOUS LEUKEMIA**
MIT PSORALEN KONJUGIERTE METHYLPHOSPHONAT-OLIGONUKLEOTIDE ALS THERAPEUTISCHE WIRKSTOFFE FÜR CHRONISCHE MYOLOGENE LEUKÄMIE
OLIGONUCLEOTIDES DE METHYLPHOSPHONATE CONJUGUES AVEC DU PSORALENE UTILISES EN TANT QU'AGENTS THERAPEUTIQUES CONTRE LA LEUCEMIE MYELOGENE CHRONIQUE

(30) Priority: 09.08.1990 US 565299
(43) Date of publication of application: 26.05.1993
(73) Proprietor: GENTA INCORPORATED, San Diego, CA 92121 (US)
(72) Inventor: VAGHEFI, Moretza, M., San Diego, CA 92130 (US); REYNOLDS, Mark, A., San Diego, CA 92130 (US); ARNOLD, Lyle, J., Jr., San Diego, CA 92117 (US)
(74) Representative: Hansen, Bernd, Dr. Dipl.-Chem.
(86) International application number: US9105690
(87) International publication number: WO9202641

(56) References cited:
- WO-A-89/02439
- WO-A-89/02896
- WO-A-92/02532
- US-A- 4 999 290
- SCIENCE vol. 245, 8 September 1989, LANCASTER, PA US pages 1107 - 1110 CARACCIOLO, D. ET AL. 'Lineage-specific requirement of c-abl function in normal hematopoiesis'
- Molecular and Cellular Biology, Volume 7, issued August 1987, S. COLLINS et al. , "Expression of bcr and bcr-abl Fusion Transcripts in Normal and Leukemic Cells", pages 2870-2876, see figure 1 and discussion.
- Biochemistry, Volume 27, issued 1988, J.M. KEAN et al., "Photochemical Cross- Linking of Psoralen-Derivatized Oligonucleoside Methylphosphonates to Rabbit Globin Messenger RNA", pages 9113-9121, see entire document.
- Anti-Cancer Drug Design, Volume 2, issued 1987, P.S. MILLER et al., "A New Approach to Chemotherapy Based on Molecular Biology and Nucleic Acid Chemistry: Matagen (masking tape for gene expression)", pages 117-128, see entire document.

## Description

### Background of the Invention

Psoralen-conjugated methylphosphonate oligomers have been reported to be capable of cross-linking to complementary sequences on single-stranded DNA and RNA in a sequence-specific manner (P.S. Miller et al., Biochemistry, 1988, vol. 27, p. 3197; Biochemistry, 1988, vol. 27, p. 9113; Nucleic Acids Res., 1988, vol. 16, p. 10697; Bioconjugate Chemistry, 1990, vol. 1, p. 82). The synthetic route for producing these compounds consists of reacting either 3-[(2-aminoethyl) carbamoyl] psoralen or 4'-[[N-(aminoethyl)amino]methyl]-4,5',8-trimethylp-soralen with a 5'-phosphorylated form of a methylphosphonate oligomer in the presence of a water soluble carbodiimide. This results in a phosphoramidate linkage between the psoralen moiety and the oligomer. Because of the nature of the chemistry employed by the methods of Miller et al., attachment of psoralen was only reported at the 5'-end of the oligomer.

Cross-linking of psoralen-conjugated methylphosphonate oligomers to target DNA or RNA occurs during irradiation at 365 nm. (For a review, see G.D. Cimino et al., Ann. Rev. Biochem., 1985, vol. 54, p. 1151). Briefly, the 4',5' (furan side) and/or 3,4 (pyrone side) carbon double bonds of the psoralens are capable of undergoing a cycloaddition reaction with pyrimidines to generate a cyclobutane linkage. These bonds are reversible under irradiation at 260 nm.

Psoralen conjugates of normal phosphodiester oligonucleotides have also been described. A phosphoramidite reagent which is an analog of 4'-hydroxymethyl-4,5',8-trimethylpsoralen has been developed which enables coupling to the 5'-end of an oligomer during automated synthesis (U. Pieles and U. Englisch, Nucleic acids Res., 1989, vol. 17, p. 285). An analog of 4'-(aminomethyl)-4,5',8-trimethylpsoralen has been synthesized with a cleavable disulfide linkage which terminates in a primary amine for coupling to a 5'-phosphorylated oligomer using a water soluble carbodiimide (J. Teare and P. Wollenzein, Nucleic Acids Res., 1990, vol. 18, p. 855). Both of these approaches have a drawback in that they only permit attachment of psoralen to the 5'-end of an oligomer. A psoralen analog has also been attached to the C8-position of deoxyadenosine and converted into a phosphoramidite reagent for incorporation into an oligonucleotide (U. Pieles et al., Nucleic Acids Res., 1989, vol. 17, p. 8967). This latter reagent only enables attachment at adenine positions and may interfere with base-pairing.

The first specific chromosome abnormality to be associated with cancer was the Philadelphia Chromosome (Ph¹), named for the city in which it was discovered. This small chromosome has been reported to be present in the leukemic cells of at least 90 percent of patients with chronic myelogeneous leukemia (CML), an invariably fatal cancer involved uncontrolled multiplication of myeloid stem cells. This chromosome abnormality has been reported in some patients having other types of leukemia, such as ANLL (acute nonlymphocytic leukemia) and ALL (acute lymphocytic leukemia). Ph¹ is derived from chromosome 22 by a reciprocal translocation involving chromosome 9 (wherein a portion of the long arm of chromosome 22 is translocated to chromosome 9 while a small fragment from the tip of the long arm of chromosome 9 is translocated to chromosome 22. Thus, two abnormal chromosomes are produced (Ph¹ and 9q⁺). Two chromosomal breaks are required to generate Ph¹. One occurs in the region of chromosome 22 called the breakpoint cluster region ("bcr") which lies within the bcr gene. The second break occurs in chromosome 9 in the 5' half of the abl gene. When chromosomes 9 and 22 fuse to give Ph¹, the 5' half of the bcr gene ends up on the 5' side of abl, with the two genes lying in the same transcriptional orientation. A large precursor RNA encompassing both genes is spliced so the 5' exons of the bcr gene are joined to a specific exon in the middle of c-abl. The abl gene has amino acid sequence homology to the tyrosine kinase family of oncogenes. The tyrosine kinase activity present in the product of the normal proto-oncogene, c-abl, is down-regulated by a peptide sequence normally found at the N-terminus. Removal of this peptide and its replacement with a piece of bcr peptide locks the enzyme in the active form. P210, the bcr-abl fusion protein found in CML cells, has detectable tyrosine kinase specific kinase activity. It has been postulated that the bcr gene plays some role in activating abl. In addition, it has been suggested that the fusion of bcr to abl may cause the aberrant abl fusion protein to be over-expressed and, thus, may participate in the cancerous transformation of such cells. Mice infected with a retrovirus encoding the P210^{bcr/abl} protein were found to develop a myeloproliferative syndrome closely resembling the chronic phase of human chronic myelogenous leukemia (CML); thus, suggesting that P210^{bcr/abl} expression can induce CML. (Daley, G.Q., et al., Science 247:824-830 (1990)).

US patent 4 737 454 discloses a fast photochemical method of labelling nucleic acids for detection purposes in hybridisation assays. Psoralen is attached to biotin using a biotin-hydroxysuccinimide reagent.

US-4 874 853 discloses synthetic oligonucleotides useful in the diagnosis of chronic myelogenous leukaemia. The synthetic oligonucleotides have sequences overlapping portions of the sequences of bcr/abl which are useful as probes.

US-4 835 263 discloses compounds containing an oligonucleotide sequence bonded to an intercalating agent. The patent teaches that hybridising oligo-nucleotides comprising alkyl phosphonate may be labelled with psoralen.

WO 86/00074 discloses chemically marked nucleic acid inhibition of mRNA expression, by anti-sense psoralen. Inhibition of mRNA expression by methyl phosphonate-containing oligo-nucleotides is also disclosed. This is indicated to occur in both cell-free systems and in cell cultures.

### Summary of the Invention

In one aspect, the present invention is directed to a reagent which enables an analog of 4'-aminomethyl, 4,5',8-trimethylpsoralen to be conveniently coupled with any second molecular species possessing a reactive primary amino group. We have found this reagent to be particularly suited for the production of psoralen-conjugated oligomers, including phosphate diester oligomers and especially alkyl- and aryl-phosphonate oligomers. Preferred alkyl- and aryl-phosphonate oligomers include methylphosphonate oligomers. In a preferred embodiment of the present invention, these oligomers have been modified to contain a reactive amine group using non-nucleotide reagents such as those described herein and in our commonly-assigned, co-pending patent application, "Improved Non-nucleotide-Based Linker Reagents for Oligomers.", WO 92/02532.

In one aspect, the psoralen labeling reagent of the present invention comprises an N-hydroxysuccinimide (NHS) activated ester moiety attached via a sidechain to the amino group of 4'-aminomethyl-4,5',8-trimethylp-soralen. NHS-activated ester functionalities have been reported for attaching chemical moieties to biomolecules which contain primary amines. The present invention also provides novel methods of synthesis for the novel psoralen reagent of the present invention.

The present invention also provides methods for carrying out the coupling reaction between this psoralen reagent and primary amine-linker modified alkyl- and aryl-phosphonate oligomers. Once coupled, the resulting psoralen-conjugated oligomers is readily isolated from unreacted oligomer using reverse-phase high performance liquid chromatography.

The present invention provides a method of attaching psoralen to oligomers which are advantageous in comparison to existing methods which employ carbodiimides as condensing agents, since carbodiimides have been disadvantageously shown to undergo side reactions with nucleotide bases which results in undesired side products. (See, Ghosh, S.S., et al., Nucl. Acids Res. 15(13):5353-5372 (1987).

Accordingly, one aspect of the present invention is directed to interfering with expression of P210^{bcr/abl} by hybridizing a psoralen-conjugated oligomer to the bcr-abl mRNA followed by cross-linking of the oligomer to the mRNA may prevent P210^{bcr/abl}-mediated transformation and induction of CML-states. Oligomers complementary to a region of the normal abl gene may be useful in down-regulating P210 tyrosine kinase activity in CML cells, as well as oligomers complementary to bcr/abl and, in particular, the bcr/abl junction. Sequences for these oligomers complementary to the bcr/abl region of the Philadelphia chromosome's chimeric bcr/abl mRNA have been synthesized. Conjugatic- of psoralen labelled oligomers complementary to that of mRNA may enhance the inhibitory effects of these oligonucleotides on the translation of this mRNA and on expression of its corresponding P210 tyrosine kinase. This inhibition is intended to down-regulate abnormal cells in patients with chronic myelogenous leukemia. In one preferred aspect, oligomers complementary to bcr/abl, preferably the bcr/abl junction, and, which selectively hybridize to the chimeric bcr/abl mRNA are selected. Such oligomers have a sequence of sufficient length that they will hybridize only to the chimeric bcr/abl mRNA and not to the normal abl mRNA sequence.

### Definitions

As used herein, the following terms have the following meanings, unless expressly stated to the contrary:

The term "nucleoside" includes a nucleosidyl unit and is used interchangeably therewith.

The term "nucleotide" refers to a subunit of a nucleic acid consisting of a phosphate group, a 5 carbon sugar and a nitrogen containing base. In RNA the 5 carbon sugar is ribose. In DNA, it is a 2-deoxyribose. The term also includes analogs of such subunits.

The term "nucleotide multimer" refers to a chain of nucleotides linked by phosphodiester bonds, or analogs thereof.

An "oligonucleotide" is a nucleotide multimer generally about 10 to about 100 nucleotides in length, but which may be greater than 100 nucleotides in length. They are usually considered to be synthesized from nucleotide monomers, but may also be obtained by enzymatic means.

A "deoxyribooligonucleotide" is an oligonucleotide consisting of deoxyribonucleotide monomers.

A "polynucleotide" refers to a nucleotide multimer generally about 100 nucleotides or more in length. These are usually of biological origin or are obtained by enzymatic means.

A "nucleotide multimer probe" is a nucleotide multimer having a nucleotide sequence complementary with a target nucleotide sequence contained within a second nucleotide multimer, usually a polynucleotide. Usually the probe is selected to be perfectly complementary to the corresponding base in the target sequence. However, in some cases it may be adequate or even desirable that one or more nucleotides in the probe not be complementary to the corresponding base in the target sequence.

A "non-nucleotide monomeric unit" refers to a monomeric unit which does not significantly participate in hybridization of a polymer. Such monomeric units must not, for example, participate in any significant hydrogen bonding with a nucleotide, and would exclude monomeric units having as a component, one of the 5 nucleotide bases or analogs thereof.

A "nucleotide/non-nucleotide polymer" refers to a polymer comprised of nucleotide and non-nucleotide monomeric units.

An "oligonucleotide/non-nucleotide multimer" is a multimer generally of synthetic origin having less than 100 nucleotides, but which may contain in excess of 200 nucleotides and which contains one or more non-nucleotide monomeric units.

A "monomeric unit" refers to a unit of either a nucleotide reagent or a non-nucleotide reagent of the present invention, which the reagent contributes to a polymer.

A "hybrid" is the complex formed between two nucleotide multimers by Watson-Crick base pairing s between the complementary bases.

The term "oligomer" refers to oligonucleotides, nonionic oligonucleoside alkyl- and aryl-phosphonate analogs, phosphorothiorate analogs of oligonucleotides, phosphoamidate analogs of oligonucleotides, neutral phosphate ester oligonucleotide analogs, such as phosphotriesters and other oligonucleotide analogs and modified oligonucleotides, and also includes nucleotide/non-nucleotide polymers. The term also includes nucleotide/non-nucleotide polymers wherein one or more of the phosphorous group likages between monomeric units has been replaced by a non-phosphorous linkage such as a formacetal linkage or a carbamate linkage.

The term "alkyl- or aryl-phosphonate oligomer" refers to nucleotide oligomers (or nucleotide/non-nucleotide polymers) having internucleoside (or intermonomer) phosphorus group linkages wherein at least one alkyl- or aryl- phosphonate linkage replaces a phosphodiester linkage.

The term "methylphosphonate oligomer" (or "MP-oligomer") refers to nucleotide oligomers (or nucleotide/non-nucleotide polymer) having internucleoside (or intermonomer) phosphorus group linkages wherein at least one methylphosphonate internucleoside linkage replaces a phosphodiester internucleoside linkage.

In some of the various oligomer sequences listed herein "p" in, e.g., as in ApA represents a phosphate diester linkage, and "p" in, e.g., as in CpG represents a methylphosphonate linkage. Certain other sequences are depicted without the use of p or p to indicate the type of phosphorus diester linkage. In such occurrances, A as in ATC indicates a phosphate diester linkage between the 3'-carbon of A and the 5' carbon of T, whereas **A**, as in **A**TC or **ATC** indicates a methylphosphonate linkage between the 3'-carbon of A and the 5'-carbon of T or **T**.

The term "non-adverse conditions" describes conditions (of reaction or synthesis) which do not substantially adversely the polymer skeleton and its sugar, base, linker-arm and label components, nor the monomeric reagents. One skilled in the art can readily identify functionalities, coupling methods, deprotection procedures and cleavage conditions which meet these criteria.

The term "deblocking conditions" describes the conditions used to remove the blocking (or protecting) group from the 5'-OH group on a ribose or deoxyribose group.

The term "deprotecting conditions" describes the conditions used to remove the protecting groups from the nucleoside bases.

The term "chimeric mRNA" refers to a messenger RNA which is a transcript of portions of two or more gene sequences which would not normally be adjacent, but which may have been brought together by occurrences such as chromosome translocation, recombination, and the like.

The term "tandem oligonucleotide" or "tandem oligomer" refers to an oligonucleotide or oligomer which is complementary to a sequence 5' or 3' to a target nucleic acid sequence and which is co-hybridized with the oligomer complementary to the target sequence. Tandem oligomers may improve hybridization of these oligomers to the target by helping to make the target sequence more accessible to such oligomers, such as by decreasing the secondary structure of the target nucleic acid sequence.

### Brief Description of the Drawings

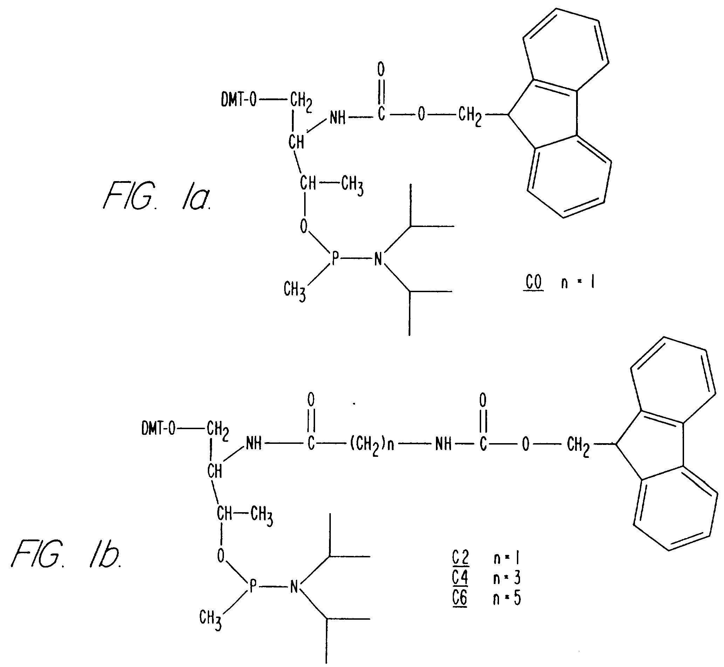

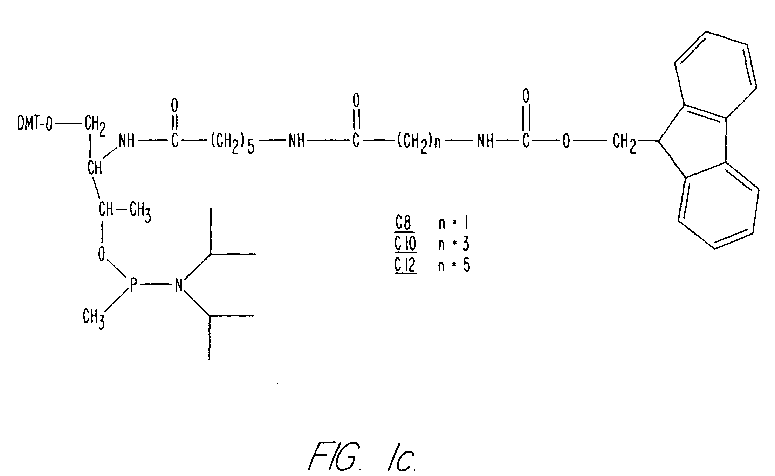

Figures 1A, 1B and 1C depict the formulas of non-nucleotide reagents having Fmoc-protected linker arms which may be conjugated to the psoralen reagents of the present invention.

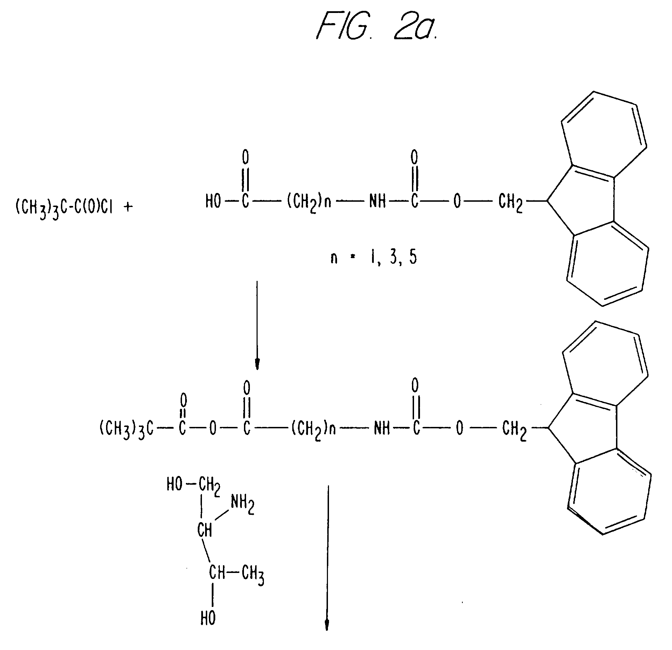

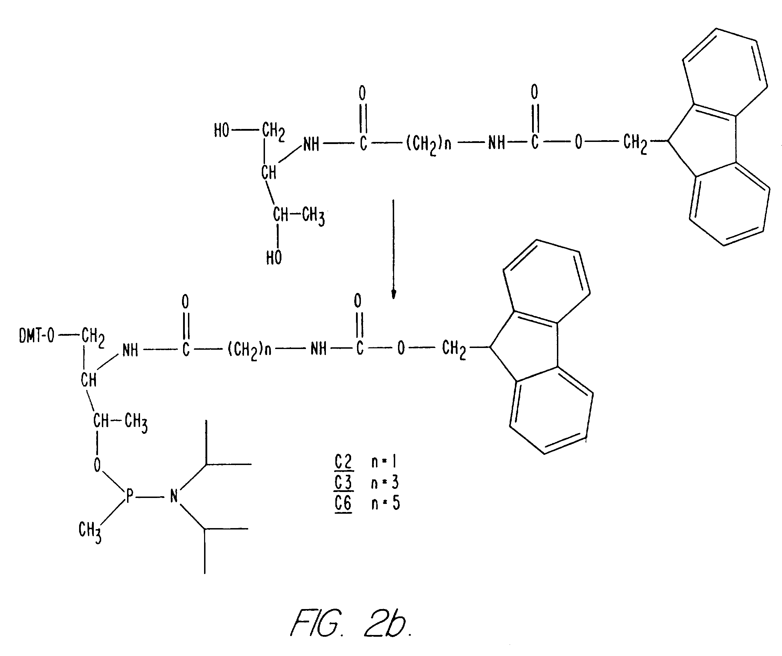

Figure 2 depicts a synthetic scheme for preparing the non-nucleotide reagents of Figure 1B.

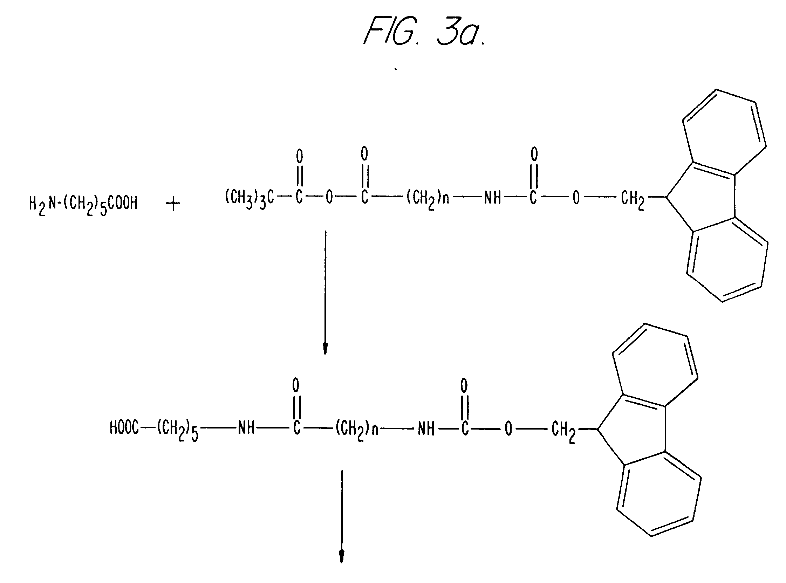

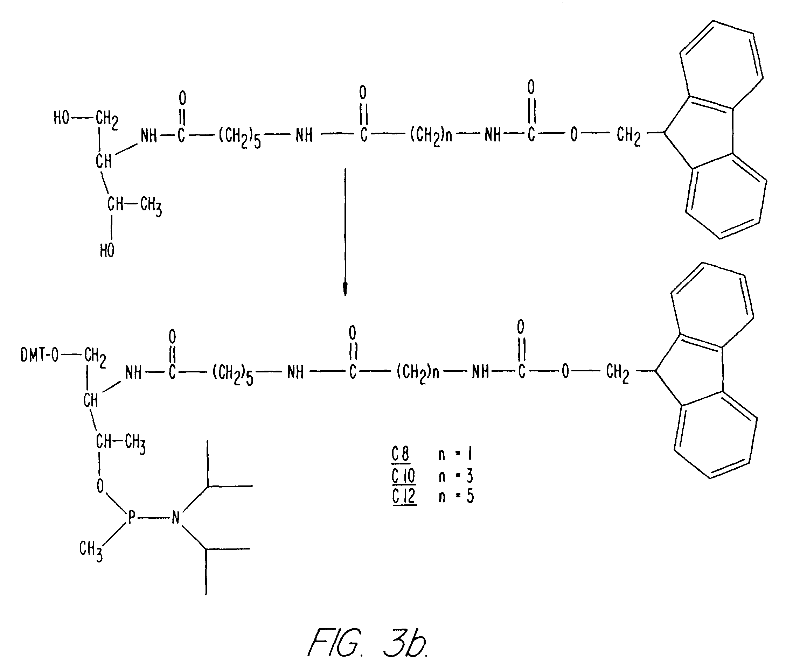

Figure 3 depicts a synthetic scheme for preparing the non-nucleotide reagents of Figure 1C.

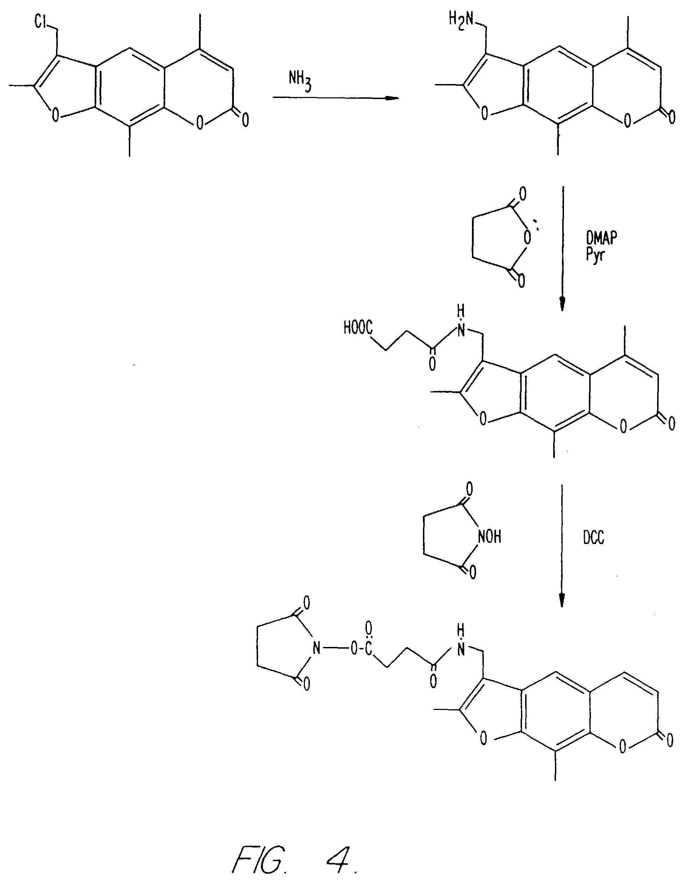

Figure 4 depicts a synthetic scheme for a preferred psoralen reagent of the present invention.

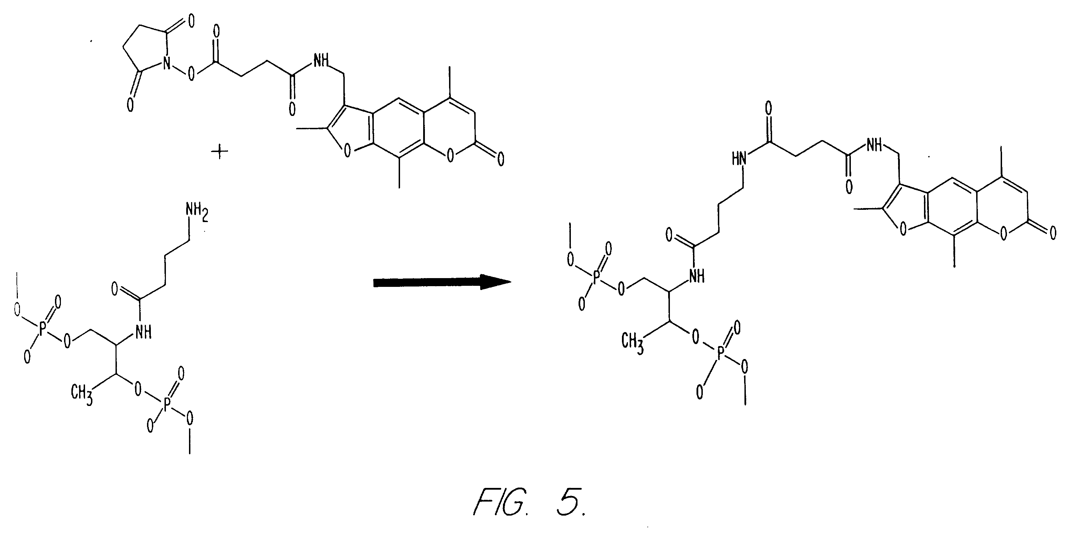

Figure 5 depicts a synthetic scheme for the conjugation of a preferred psoralen reagent to a non-nucleotide - agent.

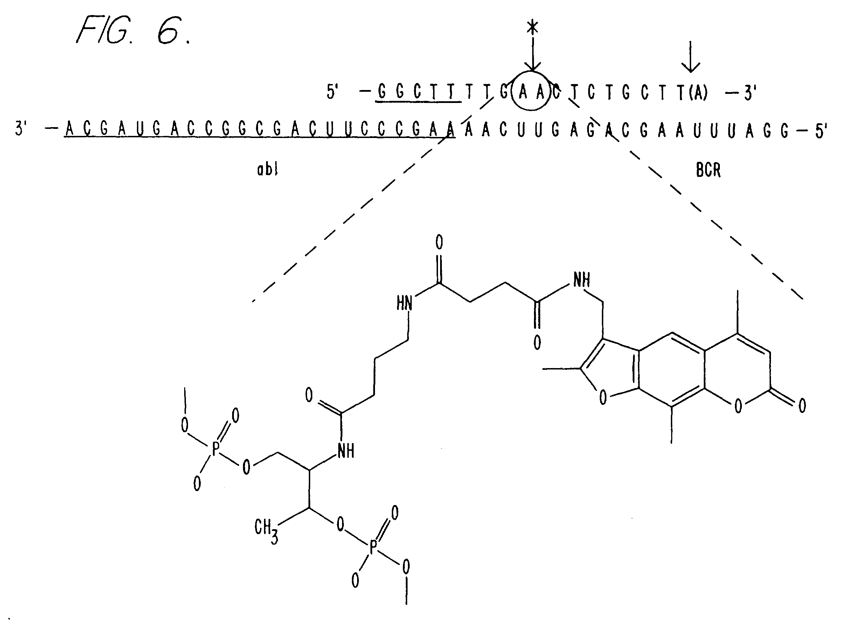

Figure 6 depicts the nucleotide sequence of oligomers incorporating psoralen conjugated non-nucleotide monomeric units complementary to a portion of a bcr/abl mRNA.

### Detailed Description of the Invention Preferred Psoralen Reagents

According to the present invention, preferred reagents for conjugating a psoralen analog moiety to an oligomer comprise compounds of the formula: 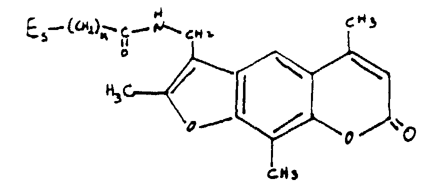 wherein k is an integer from 0 to 12 and Es is a moiety capable of coupling with a nucleophilic moiety. For example, Es may comprise an activated ester with a leaving group which is readily displaced by a second nucleophilic moiety. Preferred are compounds where k is 2 to 6. Preferred Es groups include N-hydroxysuccinimide activated esters, haloacetyls, isothio-cyanates, maleiimides and the like. Especially preferred are compounds where k is 2. One particularly preferred Es group comprises:

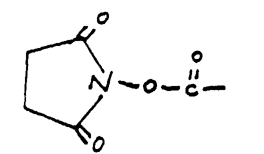

These psoralen reagents of the present invention may be conveniently prepared according to the procedures described in Examples 1 to 3. In one preferred aspect, these psoralen reagents may conveniently couple to nucleophilic non-nucleotide reagent modified oligomers under conditions which minimize side reactions on nucleotide bases, for example, in contrast to the use of conventional water-soluble carbodiimides.

### Preferred Oligomers

Preferred oligomers to be conjugated with the psoralen reagents of the present invention include oligomers which have been modified to incorporate one or more non-nucleotide monomers using the non-nucleotide reagents such as those described in Examples 4 to 11 and in the commonly assigned and co-pending U.S. Patent Application "Improved Non-Nucleotide-Based Linker Reagents for Oligomers." Particularly preferred oligomers include alkyl- and aryl- phosphonate nucleotides which incorporate at least one such non-nucleotide monomer. Especially preferred alkyl- and aryl-phosphonate oligomers include methylphosphonate oligomers.

Such alkyl- and aryl-phosphonate oligomers advantageously have a nonionic phosphorus backbone which allows better uptake of oligomers by cells. Also, the alkyl- and aryl- phosphonate intermonomeric linkages of such alkyl- and aryl-phosphonate oligomers are advantageously resistant to nucleases.

Where the oligomers comprise alkyl- or aryl-phosphonate oligomers, it may be advantageous to incorporate nucleoside monomeric units having modified ribosyl moieties. The use of nucleotide units having 2'-O-alkyl- and in particular 2'-O-methyl-, ribosyl moieties, in these alkyl or aryl phosphonate oligoemrs may advantageously improve hybridization of the oligomer to its complementary target nucleic acid sequence.

Preferred non-nucleotide reagents for use with these psoralen reagents comprise non-nucleotide monomeric units in which the skeleton has a backbone of up to 2 to about 10 carbon atoms in which said backbone comprises at least one asymmetric carbon which remains chirally pure upon being coupled into a nucleotide/non-nucleotide polymer. Skeletons having backbones of about three carbons are preferred, in part, because such backbones resemble the three-carbon spacing of deoxyribose groups.

One such preferred group of non-nucleotide reagents comprise chirally pure non-nucleotide reagents which when incorporated in an oligomer comprise a chirally pure non-nucleotide monomeric unit of the formula: 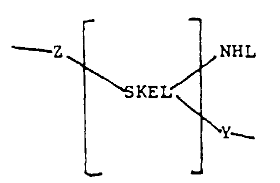 wherein SKEL comprises a chirally pure non-nucleotide skeleton of from about 1 to about 20 carbon atoms, wherein -NHL, Y and Z are covalently linked to a carbon atom of SKEL, L is a ligand, Y is -CH₂-, -O-, -S- or -NH- and Z is -O-, -S- or -NH-. Preferably SKEL further comprises a backbone of about 1 to about 10 carbon atoms separating Y and Z. Examples of non-nucleotide monomeric units incorporating these preferred SKEL groups include: 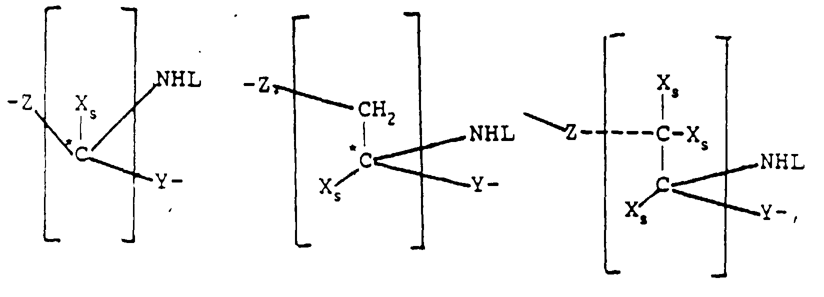 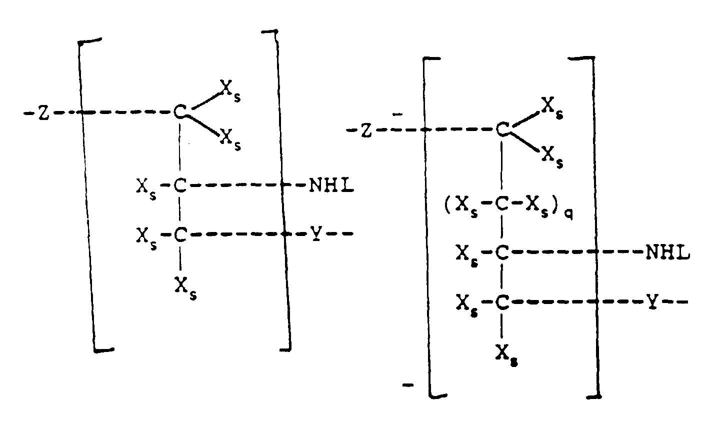 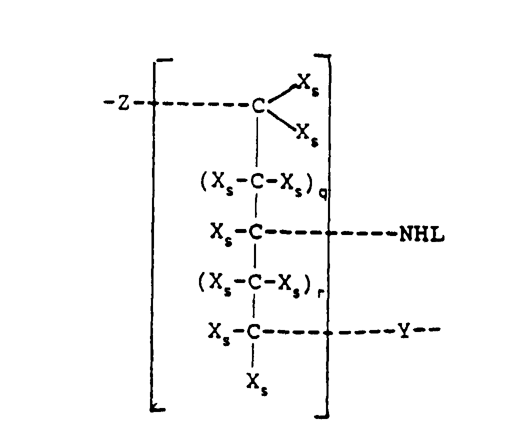 wherein the Xₛ groups are independently selected from hydrogen or alkyl and may be the same or different, and q and r are independently selected integers from 0 to 10.

Thus, in one embodiement, these preferred non-nucleotide reaaents may be represented by the general formula: 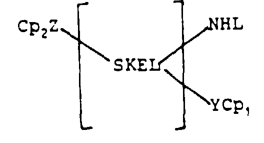 wherein -Y-Cp₁ is a first coupling group, -Z-Cp₂ is a blocked second coupling group, wherein L, Y and Z are as defined above and
(a) the first coupling group, -YCp₁ is selected from: 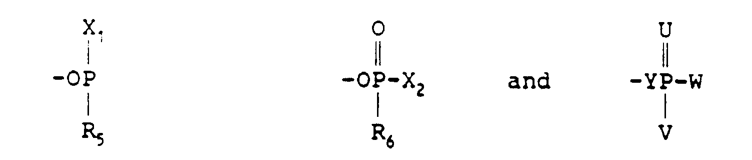 wherein X₁ is halogen or substituted amino; X₂ is halogen, amino, or substituted amino, or O⁻; R₅ is alkyl, optionally substituted alkoxy or optionally substituted aryloxy; and R₆ is alkyl, optionally substituted alkoxy or optionally substituted aryloxy, or if X₂ is O⁻, optionally hydrogen; U is oxygen, sulfur or imino, W is alkyl, aryl, alkoxy, aryloxy, alkylthio, arylthio, S⁻, O⁻, amino or substituted amino, and V is alkoxy, alkylthio, amino or substituted amino.
(b) blocked second coupling group, -ZCp₂, wherein Cp₂, is a blocking group cleavable under deblocking conditions to recover the second coupling group -XH wherein Z is -O-, -NH- or -S-.

Since preferred are non-nucleotide reagents which are capable of forming alkyl- or aryl-phosphonate, and in particular methylphosphonate, diester linkages between monomeric units, especially preferred non-nucleotide reagents include those wherein the first coupling group, - YCp₁, is selected from 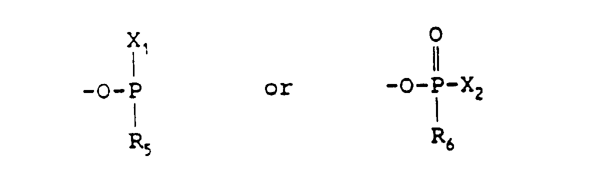 wherein X₁ is chloro or secondary amino and R₅ is alkyl; X₂ is substituted amino, halogen or O⁻ and R₆ is alkyl.

The ligand moiety, L is preferably selected from a functional moiety or from a protected linking arm which can be deprotected under non adverse conditions so as to be capable of then linking with a functional moiety (under non-adverse conditions).

In one preferred aspect of the present invention, L comprises a protecting group, Pr, or protected linker arm which can be deprotected under non-adverse conditions so as to be capable of then linking with a functional moiety, including a cross linking agent such as psoralen, or a drug carrier molecule. Preferred linker arms include those having one of the following formulas: 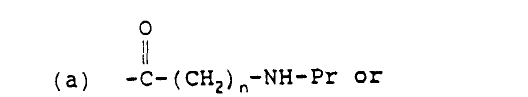 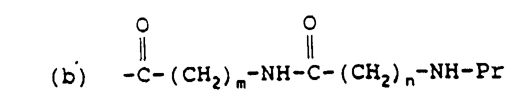 wherein n and m are independently integers between 1 and 15, preferably between 1 and 5, and Pr is a protecting group removable under non-adverse conditions.

One group of particularly preferred non-nucleotide reagents has a skeleton derived from the amino acid threonine. These preferred reagents comprise a 3-carbon backbone having two asymmetric carbons, each of which remains chirally pure when incorporated in a nucleotide/non-nucleotide polymer. In addition, these reagents having threonine-derived backbones advantageously have a primary hydroxyl and a secondary hydroxyl, which due to their differing reactivities allow selectivity and high yields in the subsequent protection, deprotection, blocking, deblocking and derivatization steps. In one preferred embodiment of the present invention, the first coupling group is associated with the secondary hydroxyl group and the second coupling group is associated with the primary hydroxyl.

Thus, according to an especially preferred aspect of the present invention, the threonine-based non-nucleotide reagents have the following formula: 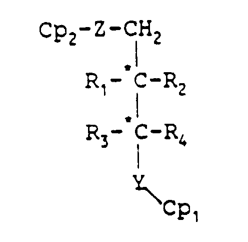 wherein *C denotes an asymmetric carbon which is chirally pure, and wherein one of R₁ and R₂ is hydrogen and the other is -NH-L where L is a ligand moiety as hereinafter defined; one of R₃ and R₄ is hydrogen and the other is lower alkyl of about 1 to about 10 carbon atoms, -Y-Cp₁ is a first coupling group, and -ZCp₂ is a blocked second coupling group, wherein:
(a) The first coupling group, -YCp₁, wherein Y is -CH₂-, -S-, -NH-, or -O- is selected from 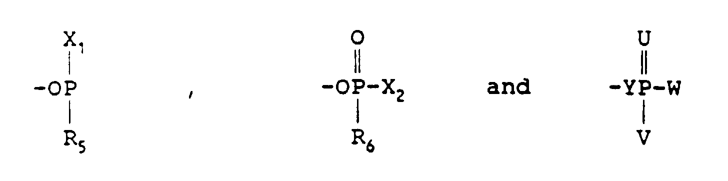 wherein X₁ is halogen or substituted amino; X₂ is halogen, amino, or substituted amino, or O⁻; R₅ is alkyl, optionally substituted alkoxy or optionally substituted aryloxy; and R₆ is alkyl, optionally substituted alkoxy or optionally substituted aryloxy, or if X₂ is O⁻, optionally hydrogen; U is oxygen or sulfur, W is alkyl, aryl, alkoxy, alkylthio, aryloxy, arylthio, O⁻, S⁻, amino or substituted amino; and V is alkoxy, alkylthio, amino or substituted amino; and
(b) blocked second coupling group, -ZCp₂, wherein Cp₂, is a blocking group cleavable under deblocking conditions to recover the second coupling group -ZH wherein Z is -O-, -NH- or -S-.

The ligand moiety, L is preferably selected from a functional moiety or from a protected linking arm which can be deprotected under non-adverse conditions so as to be capable of then linking with a functional moiety (under non-adverse conditions).

Preferred non-nucleotide reagents for use with the psoralen reagents of the present invention include those having C2 and C4 linker arms. When the psoralen-conjugated non-nucleotide reagent is incorporated in the interior sequence of an oligomer, C2 and C4 non-nucleotide reagents with linker arms appear to afford enhanced cross-linking. When a psoralen-conjugated non-nucleotide reagent is incorporated at the 3'-end of the oligomer or one or so monomeric units before the 3'-end, non-nucleotide reagents having C4 linker arms are advantageously effective in cross-linking with the complementary nucleic acid.

Preferred oligomers include those where one of the psoralen reagents of the present invention has reacted with the terminal amine of a non-nucleotide monomeric unit (after deprotection of the amine) to give a psoralen-conjugated oligomer. In view of enhanced cross-linking between the psoralen-conjugated non-nucleotide monomeric unit and the complementary target nucleic acid, preferred are oligomers wherein the psoralen conjugated non-nucleotide monomeric unit is incorporated next to or in close proximity to a thymidine, uridine or cytidine base of the complementary strand or, correspondingly, to an adenine or guanine base of the anti-sense strand so that it can conveniently react with a thymidine or uridine base on the complementary nucleic acid strand. More preferred is for the non-nucleotide monomeric unit to be located between an adenine and thimidine base (5'-3') of the anti-sense strand.

Thus, preferred are oligomers having non-nucleotide monomeric units of one of the above-noted structures, in which after reaction with the appropriate psoralen reagent, L is selected from: 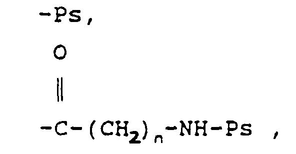 and 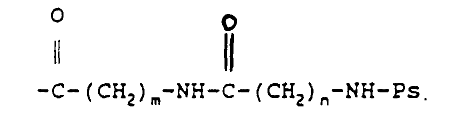 wherein n and m are independently integers from about 1 to about 15, preferably from 1 to 5, and Ps comprises a psoralen moiety. Preferred are psoralen moieties of the formula: 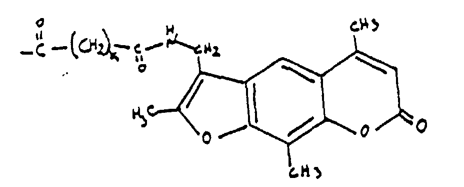 wherein k is an integer from 0 to 12. Especially preferred are psoralen moieties where k is 2.

Preferred are oligomers which comprise from about 6 to about 25 nucleotides, more preferably from about 12 to about 20 nucleotides. Such oligomers may include from about 1 to about 5 independently selected non-nucleotide monomeric units. Although oligomers which comprise more than about 20 nucleotides may be used, where complementarity to a longer sequence is desired, it may be advantageous to employ shorter tandem oligomers to maximize solubility and transport across cell membranes while competing for the development of a secondary structure of the target nucleic acid, such as a mRNA.

### Utility

According to the present invention, oligomers which incorporate psoralen-conjugated non-nucleotide monomeric units may be synthesized which are complementary to a selected target nucleic acid sequence, either RNA or DNA. After hybridizing the psoralen-conjugated oligomer to the target nucleic acid, the psoralen moiety is caused to cross-link the complementary target strand by reacting with a pyrimidine base of the complementary nucleic acid target in a cycloaddition reaction. Such cross-linking of oligomer to target nucleic acid interferes with the transcription or translation functions of the nucleic acid. For example, if the target nucleic acid is a messenger RNA, cross-linking of oligomer to mRNA will interfere with its translation and, thus, expression of the polypeptide it codes for. Moreover, such cross-linking of oligomer to mRNA potentiates the interfering or inhibitory effect of hybridizing an anti-sense oligomer to the complementary target sequence.

Thus, the reagents of the present invention can be useful in methods of potentiating the effect of anti-sense oligomers in interfering with and/or inhibiting nucleic acid function by hybridizing anti-sense psoralen-conjugated oligomers to a complementary target sequence and causing the psoralen moieties to cross-link with the complementary target nucleic acid sequence. In particular, these psoralen-conjugated oligomers may be used to inhibit synthesis of a protein coded by a certain mRNA by hybridizing the oligomer to the mRNA and then causing the psoralen to cross-link with the mRNA to interfere with its translation.

Accordingly, the present invention provides the use of an oligomer, which is complementary to a portion of the bcr/abl region and effective to prevent expression of P210^{bcr/abl}, in the manufacture of a medicament effective against chronic myelogenous leukaemia or isolated cells thereof.

The present invention also provides the use of an oligomer, which is complementary to a nucleic acid coding for a protein of the abl gene or mRNA, effective to decrease expression of tyrosine kinase in the manufacture of a medicament effective against chronic myelogenous leukaemia or isolated cells thereof.

In these applications, a psoralen-conjugated oligomer is synthesized which is complementary to the junction of the bcr and abl genes of the chimeric mRNA present in cells carrying the Philadelphia chromosome. The reciprocal translocation produces the (abnormal) Philadelphia chromosome in which the coding sequence for the bcr gene (from Chromosome 9) is juxtaposed with the coding sequence for the c-abl gene (from Chromosome 22). The spliced bcr/abl genes produce a chimeric mRNA which codes for a P210^{bcr/abl} protein. The presence cf the Philadelphia chromosome and the resulting expression of the P210^{bcr/abl} protein has been found to be associated with chronic myelogenous leukemia (CML) in humans and to induce CML-like states in animals (mice). A psoralen-conjugated oligomer complementary to the bcr/abl junction will hybridize only to the chimeric mRNA. If the psoralen-conjugated oligomer is first allowed to hybridize to the bcr/abl chimeric mRNA and then to cross-link with the mRNA and it will therefore interfere with expression with the P210^{bcr/abl} protein.

The translocation which results in the bcr/abl fusion cuts off the 5'-end of the c-abl gene coding for tyrosine kinase. Due to removal of the 5'-abl sequence, this translocation results in no down regulation of the enzyme. Moreover, due to the presence of a portion of the bcr promoter, the P210 protein may be over-expressed. Oligomers complementary to the normal abl region, may be used to event over-expression of P210.

To assist in understanding the present invention, the following examples are included which describe the results of a series of experiments.

### Examples

### Example 1

### Preparation of 4'-Aminomethyl-4 5',8-Trimethylpsoralen

4'-Chloromethyl-4,5',8-trimethylpsoralen was synthesized according to the procedure of Isaacs et al. (Biochemistry, 16, (1977), 1058-1064). 330 mg of this compound was dissolved in 100 ml of anhydrous acetonitrile and cooled to -10°C. Dry ammonia was bubbled into this solution until saturation. The cooling bath was removed and the reaction mixture was allowed to warm up to room temperature and then stirred overnight. The solvent was evaporated under reduced pressure and the residue was dissolved in 80 ml tetrahydrofuran/acetone (3:1) and filtered. The filtrate was dried to yield 300 mg of the above-identified product (a quantitative yield).

### Example 2

### Preparation of 4'-Succiniamidomethyl-4,5',8-Trimethylpsoralen

4'-Aminomethyl-4,5',8-trimethylpsoralen (300 mg) was dried by co-evaporation with anhydrous pyridine and dissolved in 30 ml of dry pyridine. To this solution 500 mg of succinic anhydride and 50 mg dimethylaminopyridine was added and stirred at room temperature for 3 hours. The reaction was monitored by TLC. Pyridine was evaporated under reduced pressure and the residue was dissolved in 30 ml dichloromethane and 5 ml methanol was added. The product started to crystallize at this time; an extra 30 ml dichloromethane was added and the crystals were collected after 2 hours to give 370 mg of the above-identified product.

### Example 3

### Activation of the Free Carbonyl Moiety of 4'-Succinamidomethyl- 4,5' 8-Trimethylpsoralen With N-Hydroxysuccinimide

4'-Succinamidomethyl-4,5',8-trimethylpsoralen (100 mg) was dried by co-evaporation with anhydrous pyridine. The dry residue was dissolved in 20 ml of anhydrous dimethylformamide and 20 ml anhydrous dioxane. To this solution 700 mg of dry N-hydroxysuccinimide was added and 2 ml of a 20% solution of dicyclohexylcarbodiimide (DCC) in anhydrous dioxane was added. The reaction mixture was stirred at room temperature overnight. The reaction mixture was filtered and washed with 30 ml dioxane. The filtrate was evaporated to dryness and the residue was sonicated in 40 ml ethyl acetate for 5 min. The slurry precipitate was filtered to give 110 mg of the above-identified product.

### Example 4

### Reduction of L-Threonine Methyl Ester

L-Threonine methyl ester (purchased from Sigma) was reduced according to the procedure of Stanfield et al. (J. Org. Chem. (1981), 46, 4799): in a 500 ml three necked flask, 5 g of L-threonine methyl ester and 200 ml dry THF were mixed and 150 ml of 1 M solution of LiAlH₄ was added dropwise with stirring while under argon. The reaction mixture was then warmed up to the boiling temperature of THF and refluxed under argon overnight. The completion of the reaction was monitored by TLC on Silica Gel which was visualized with ninhydrin. The reaction mixture was cooled to 5-10° C and quenched with dropwise addition of 0.25 M NaOH (100 ml). The mixture was evaporated to remove over 90% of THF and the residue was diluted with 100 ml of dimethylformamide which facilitates the filtration. The mixture was then filtered through a Whatman #1 paper using aspirator vacuum. The filtrate was evaporated to dryness and the residue was purified on a flash Silica Gel column. The column was packed with dichloromethane and the product was eluted with 50% methanol in dichloromethane.

### Example 5

### Synthesis of 4-N-(9-Fluorenylmethoxycarbonyl)-4-Amino-N-Butyric Acid

Fmoc-aminobutyric acid (for C4 linker arm) was prepared according to the following procedure. (Note: other FMOC-aminocarboxylic acids are commercially available. For example, Fmoc-aminocaproic acid (for C6 linker arm) and Fmoc-glycine (for C2 linker arm) are commercially available from Bachem, Inc., Torrance, California).

A mixture of 1.8 g 4-aminobutyric acid and 1.24 g sodium hydrogen carbonate in 35 ml water/acetone (50:50) was prepared and 5 g Fmoc-succinimidyl carbonate (N-Fluorenylmethyl-succinimidylcarbonate) (Bachem) was added. The reaction mixture was stirred overnight at room temperature. The pH of the reaction mixture was adjusted to 2 by 1N HCl and the solvent was removed under reduced pressure and the residue was dissolved in 20 ml ethanol and filtered. The filtrate was evaporated to dryness and the residue was taken up in dichloromethane and filtered to give 4.8 g of pure product.

¹H NMR in DMSO-d6, 1.61 (CH₂), 2.22 (CH₂), 3.01 (CH₂-N), 4.32 (CH₂-C=O), 4.22 (NH), 7.25-7.95 (8 aromatic protons).

### Example 6

### Blocking of the Amine Moiety of Reduced L-Threonine

The amine moiety of the reduced L-threonine was coupled with a 9-fluorenylmethoxycarbonyl ("Fmoc") group using with a procedure similar to the Fmoc-aminobutyric acid preparation described above. After the overnight reaction, adjustment of the pH was not necessary. The solvent was removed and the residue was dissolved in 40 ml dichloromethane and extracted with water (2 x 50 ml). The organic phase was then dried and purified on a flash Silica Gel column. The product was eluted with 2% methanol in dichloromethane to give 3.85 g of the product.

¹H NMR 1.20 (CH₃), 2.85 (NH), 3.26 (CH), 3.48 (CH), 3.72 (OH), 7.3-7.9 (8 aromatic protons).

### Example 7

### Preparation of Fmoc-Blocked Linker Arms:

### Fmoc-Glycylamido-Caproic Acid (C8), Fmoc-4-Aminobutrylamido-Caproic Acid (C10) and Fmoc-Caproylamido-Caproic Acid (C12)

Fmoc-glycine, Fmoc-4-aminobutyric acid and Fmoc-aminocaproic acid were coupled to the aminocaproic acid in order to synthesize the above-identified C8, C10 and C12 linker arm. The desired Fmoc-amino acid (17 mmol) was dried with co-evaporation with dry pyridine (3 x 30 ml). The dried material was then dissolved in 30 ml of dry dimethylformamide and 30 ml dry tetrahydrofuran was added. The solution was cooled to 0°C and 1 equivalent (17 mmol) of N,N-diisopropylethylamine was added. While stirring, 1 equivalent of trimethylacetyl chloride was added dropwise at 0°C and stirred for 45 min. 1.2 equivalent of dry aminocaproic acid was then added and the reaction mixture was warmed up to room temperature and stirred overnight. The progress of the reaction was monitored by TLC. After the completion, the solvents were evaporated under reduced pressure. The residue was reconstituted with 50 ml water and the pH was adjusted to 2 by 1N HCl. The mixture was extracted with 100 ml of ethyl acetate and the organic phase was washed with 20 ml of water and dried (MgSO₄). The mixture was then filtered and the solvent was evaporated under reduced pressure to a volume of about 40 ml. Hexane was added dropwise to this solution until cloudiness and cleared by heating. The product was then crystallized overnight.

C8 ¹H NMR in DMSO-d6, 1.30 (CH₂), 1.39 (CH₂), 1.48 (CH₂), 2.20 (CH₂-N), 3.06 (CH₂ of FMOC), 3.58 (CH₂-COOH), 4.24 (2NH), 4.34 (CH of FMOC and CH₂ of Glycine), 7.3-7.9 (8-Aromatic protons).

C10 ¹H NMR in DMSO-d6, 1.30-1.70 (5CH₂'s), 2.07 (CH₂), 2.20 (CH-N), 3.0-3.1 (CH₂-COOH and CH₂ of FMOC), 4.26 (2NH), 4.31 (CH of FMOC), 7.3-7.9 (8-Aromatic protons).

C12 ¹H NMR in DMSO-d6, 1.2-1.5 (6CH₂'s) , 2.00 (CH₂-N), 2.18 (CH₂-N) , 2.9-3.0 (2CH₂-C=O), 4.23 (2NH), 4.31 (CH₂ of FMOC), 7.3-7.9 (8-Aromatic protons).

### Example 8

### Coupling of Reduced L-Threonine to Linker Arms

The desired linker arm (11 mmol), which was made according to Examples 5 or 7 above [Fmoc-glycine (C2), Fmoc-4-aminobutyric acid (C4), Fmoc-caproic (C6), Fmoc-glycyclamido-caproic acid (C8), Fmoc-4-aminobutyrylamidocaproic acid (C10), and Fmoc-aminocaproylamidocaproic acid (C12)], was dried with co-evaporation with pyridine (3 x 20 ml). The dry residue was dissolved in 40 ml of a mixture of anhydrous dimethylformamide and anhydrous tetrahydrofuran (1:1). The solution was cooled in an ice bath and 1 equivalent of diisopropylethylamine was added. While stirring, 1.1 equivalent of trimethylacetyl chloride was added dropwise and stirred for 45 min at 0°C. A solution of 1.5 equivalent of reduced L-threonine (Example 4 above) was added and the reaction mixture was allowed to warm to room temperature and stirred for one hour. The progress of the reaction was monitored by TLC on Silica Gel which was developed by CH₂Cl₂/CH₃OH/CH₃COOH (10:1:0.1) solvent system. After the completion of the reaction, the solvent was removed under reduced pressure and the residue was mixed with 50 ml ethyl acetate. The water soluble materials were removed by extraction with 40 ml saturated sodium bicarbonate. The organic phase was washed with 20 ml of water and dried (MgSO₄). The product was crystallized from ethyl acetate.

C2 Linker ¹H NMR in DMSO-d6, 1.03 (CH₃ of reduced L-threonine), 3.35 (OH), 3.3-3.45 (2CH), 3.91 (NH), 4.27 (other NH), 4.31 (OH), 4.34 (CH₂), 4.63 (CH₂ and CH₂ of FMOC), 7.3-7.9 (8-Aromatic protons).

C4 Linker ¹H NMR in DMSO-d6, 1.03 (CH₃ of reduced L-threonine), 1.62 (CH₂), 2.14 (CH₂), 2.91 (CH), 2.97 (CH₂), 3.3-3.5 (2CH), 3.63 (OH), 3.84 (OH), 4.23 (CH), 4.33 (CH and CH₂ of FMOC), 4.60 (NH), 6.32 (NH), 7.3-7.9 (8-Aromatic protons).

C6 Linker ¹H NMR in DMSO-d6, 1.03 (CH₃ of reduced L-threonine), 1.3-1.7 (3 CH₂'s), 2.52 (CH_{2-N}) , 3.12 (CH-C=O), 3.8-3.9 (2 OH), 4.1-4.2 (2CH), 4.41 (CH₂ of FMOC), 5.22 (NH), 6.48 (NH), 7.3-7.9 (8-Aromatic protons) .

C8 Linker ¹H NMR in DMSO-d6 major proton signals are as follows: 1.01 (CH₃ of reduced L-threonine), 1.22-1.52 (3 CH₂ of caproate), 3.62 and 3.84 (2 OH), 5.35 (NH), 6.18 (NH), 7.3-7.9 (8-Aromatic protons).

C10 Linker ¹H NMR in DMSO-d6, 1.02 (CH₃ of reduced L-threonine), 1.3-1.50 (4 CH₂'s), 3.64 (OH), 3.82 (OH), 4.64 (NH), 6.33 (NH), 6.62 (NH), 7.3-7.9 (8-Aromatic protons).

C12 Linker ¹H NMR in DMSO-d6, major proton signals for identification 1.01 (CH₃ of reduced L-threonine), 1.30-1.50 (6CH₂'s), 3.63 (OH), 3.82 (OH), 4.62 (NH), 6.31 (NH), 6.63 (NH), 7.3-7.9 (8-Aromatic protons).

### Example 9

### Dimethoxy Tritylation of the Primary Hydroxyl Moiety Of the Non-Nucleotide Reagent

The desired non-nucleotide reagent (6 mmol), which was made according to Examples 6 and 8 above, was dried by co-evaporation with dry pyridine and dissolved in 15 ml of dry pyridine. A solution of 2.2 g of dimethoxytrityl chloride in 20 ml of CH₂Cl₂/pyridine (1:1) was added dropwise with stirring. The reaction continued at room temperature for 45 min. The progress of the reaction was monitored by TLC. After the completion of the reaction it was quenched by the addition of 2 ml methanol which was stirred for 10 min. The solvents were removed under reduced pressure and the residue was dissolved in 50 ml of dichloromethane and extracted with saturated sodium hydrogen carbonate (2 x 50 ml) followed by water (30 ml). The organic phase was dried (MgSO₄) and filtered. After the evaporation of the solvent, the residue was purified with a flash column chromatography. The product was eluted with 2% methanol in dichloromethane containing 0.5% triethylamine.

C0 Linker ¹H NMR, CDCl₃, 1.18 (CH₃ of reduced L-threonine), 1.63 (CH), 2.83 (NH), 3.77 (2 CH₃ of DMT), 3.82 (CH₂ of FMOC), 5.48 (CH₂-O-DMT), 6.82-7.90 (21 aromatic protons).

C2 Linker ¹H NMR, CDCl₃, 1.18 (CH₃ of reduced L-threonine), 3.78 (2 CH₃'s of DMT), 4.35 (CH₂-O-DMT), 5.98 (NH) 6.80-7.78 (21 aromatic protons).

C4 Linker ¹H NMR, CDCl_{3,} major signals 1.18 (CH₃ of reduced L-threonine), 1.83 (CH₂), 2.28 (CH₂), 3.74 (2 CH₃ of DMT), 4.21 (OH), 4.38 (CH₂ of FMOC), 5.22 and 6.42 (2 NH), 6.80-7.65 (21 aromatic protons).

C6 Linker ¹H NMR, CDCl₃ major peaks 1.12 (CH₃ of reduced L-threonine), 1.3-1.6 (3 CH₂'s), 3.75 (2 CH₃ of DMT), 4.38 (CH₂ of FMOC), 6.80-7.90 (21 aromatic protons).

C8 Linker ¹H NMR, CDCl₃, major identifying signals were 1.12 (CH₃ of reduced L-threonine), 3.80 (2 CH₃ of DMT), 5.42 (CH₂ of FMOC), 6.18 and 6.321 (2 NH), 6.82-7.80 (21 aromatic protons).

C12 Linker ¹H NMR, CDCl₃, major identifying signals were 1.12 (CH₃ of reduced L-threonine), 3.78 (2 CH₃ of DMT), 4.59 (CH₂ of FMOC), 6.8-7.8 (21 aromatic protons).

C10 Linker ¹H NMR, CDCl₃ 1.18 (CH₃ of reduced L-threonine), 3.78 (2 CH₃ of DMT), 4.40 (CH₂ of FMOC), 6.8-7.8 (21 aromatic protons) all the CH₂ and CH (non aromatics were also accounted for but not assigned).

### Example 10

### Methylphosphinylation of the Secondary Hydroxyl Moiety of the Non-Nucleotide Reagents

A DMT blocked linker arm made according to the procedure described in Example 9 above (4 mmol) was dried by co-evaporation with dry pyridine and the residue was dissolved in 20 ml of anhydrous dichloromethane. Under closed argon atmosphere, 1.5 equivalent of diisopropylethylamine was added and 1.2 equivalent of N,N-diisopropylmethyphosphinamidic chloride [(CH₃)₂CH]₂NP (CH₃)Cl was added dropwise. The reaction was completed in 45 min. The solvent was removed under reduced pressure and the residue was purified on a flash Silica Gel column. The column was packed with ethyl acetate/hexane (1:) containing 5% triethylamine and washed with the ethyl acetate/hexane containing 1% triethylamine. The reaction mixture was then loaded on the column and the product was eluted with ethyl acetate/hexane (1:1) containing 1% triethylamine.

Other non-nucleotide reagents are prepared by coupling of the linker arm-modified reagents made according to the methods described in Example 9 with other phosphorylating agents such as N,N-diiso-propylmethyl phosphonamidic chloride [(CH₃)₂CH]₂NP(OCH₃)Cl and 2-cyanoethyl N,N-diisopropylchloro-phosphoramidite [(CH₃)₂CH]₂NP(Cl)OCH₂CH₂CN.

CO ¹H NMR, CDCl₃, 0.9-1.3 (18 protons of 6 CH₃'s), 3.11 (CH₂ of FMOC), 3.78 (2 CH₃'s of DMT), 4.42 (CH₂-O-DMT), 4.98 (NH), 6.8-7.8 (21 aromatic protons).

C4 ¹H NMR, CDCl₃, 0.9-1.2 (18 protons of 6 CH₃'s), 1.88 (CH₂), 2.21 (CH₂), 3.08 (CH₂ of FMOC), 3.80 (2 CH₃'s of DMT), 4.36 (CH₂-O-DMT), 5.16 (NH), 5.75 (NH), 6.8-7.8 (21 aromatic protons).

C6 ¹H NMR, CDCl₃, 0.9-1.2 (18 protons of 6 CH₃'s), 1.18-2.2 (4 CH₂'s), 3.07 (CH₂ of FMOC), 3.78 (2 CH₃'s of DMT), 4.42 (CH₂-O-DMT), 5.6 and 6.21 (2 NH), 6.8-7.8 (21 aromatic protons).

### Example 11

### Methylphosphinylation of the Secondary Hydroxy Moiety of a Non-Nucleotide Reagent Having a C6-Linker Arm

A 4 mmol portion of a dimethoxytrityl(DMT)-blocked non-nucleotide reagent having a C6 linker arm (prepared according to the methods described in Example 9 herein) was dried by co-evaporation with dry pyridine. The residue was dissolved in 20 ml of anhydrous dichloromethane. Under a closed argon atmosphere, 1.5 equivalents of N,N-diisopropylethylamine was added; then 1.2 equivalent of N,N-diisopropylmethylphosphonamidic chloride [(CH₃)₂CH]₂NP(Cl)OCH₃] was added dropwise. The reaction mixture was then worked up using the procedures described in Example 10 to give 3.2 mM of the above-identified product.

¹H NMR in CDCl₃, δ ppm: 1-1.5 (5 methyl and 1 methylene), 1.42 (CH₂), 1.73 and 1.73 (2 CH₂), 2.21 (CH₂-N), 3.15 (CH₂-C=O), 3.78 (2 CH₃ of DMT), 6.80-7.85 (21 aromatic protons). Other proton signals present were not assigned.

### Example 12

### Preparation of an Oligonucleotide Which Incorporates a Methoxyphosphoramidite Non-Nucleotide Reagent Having a C8 Linker Arm

A phosphate diester oligodeoxyribonucleotide was synthesized which incorporated a C8 methoxyphosphoramidite non-nucleotide reagent in the following sequence: 
5'-TTT-AAG-CAG-AGT-TCA-AAA-GCC-CTT-CAG-CG-(C8-Linker)
-T-3'
 was prepared according to the following procedure.

The C8 methoxyphosphoramidite non-nucleotide reagent (1-O-dimethoxytrityl-2-N[N'-(N"-fluorenylmethoxycarbonyl-6-aminohexanoyl)-2-aminoacetyl]-3-O-[N,N-diisopropylmethoxyphosphinyl]-2-amino-1,2-dihydroxybutane) was dissolved in dry acetone at a concentration of 100 mM and coupled into the oligonucleotide sequence using a Biosearch Model 8750 DNA synthesizer by standard phosphoramidite chemistry (M.H. Caruthers, et al., Methods of Enzymol. 154:287-313 (1985)) according to the manufacturer's recommendations. The 5'dimethoxytrityl protecting group was left on at the end of the synthesis to permit purification on a Sep-Pak™ C18 cartridge (Millipore/Waters, Bedford, MA) as described by K.M. Lo et al. (1984, Proc. Natl. Acad. Sci. USA, 81, pp. 2285-2289). During this procedure, the dimethoxytrityl protecting group was removed.

### Example 13

### Preparation of Methylphosphonate Oligonucleotides Which Incorporate Non-Nucleotide Reagents

### (a) Preparation of Methylphosphonate Oligomers

Methylphosphonate oligomers which incorporated non-nucleotide reagents of the present invention were synthesized using methylphosphonamidite monomers and non-nucleotide methylphosphonamidite non-nucleotide reagents, according to chemical methods described by P.S. Miller et al. (1983, Nucleic Acids Res., 11, pp. 6225-6242), A. Jager and J. Engels (1984, Tetrahedron Lett., 25, pp. 1437-1440), and M.A. Dorman et al. (1984, Tetrahedron, 40, pp. 95-102). Solid-phase synthesis was performed on a Biosearch Model 8750 DNA Synthesizer according to the manufacturer's recommendations with the following modifications: "G" and "C" monomers were dissolved in 1:1 acetonitrile/dichloromethane at a concentration of 100 mM. "A" and "T" monomers were dissolved in acetonitrile at a concentration of 100 mM. Non-nucleotide linker reagents were dissolved in acetonitrile at a concentration of 120 mM. DEBLOCK reagent = 2.5% dichloroacetic acid in dichloromethane. OXIDIZER reagent = 25 g/L iodine in 2.5% water, 25% 2,6-lutidine, 72.5% tetrahydrofuran. CAP A = 10% acetic anhydride in acetonitrile. CAP B = 0.625% N,N-dimethylaminopyridine in pyridine. The 5'-dimethoxytrityl protecting group was left on at the end of the synthesis to facilitate purification of the oligomers, as described below.

The crude, protected non-nucleotide reagent incorporating methylphosphonate oligomers were removed from the solid support by mixing with concentrated ammonium hydroxide for two hours at room temperature. The solution was drained from the support using an Econo-Column™ (Bio-Rad, Richmond, CA) and the support was washed five times with 1:1 acetonitrile/water. The eluted oligomer was then evaporated to dryness under vacuum at room temperature. Next, the protecting groups were removed from the bases with a solution of ethylenediamine/ ethanol/acetoni-trile/water (50:23.5:23.5:2.5) for 6 hours at room temperature. The resulting solutions were then evaporated to dryness under vacuum.

### (b) Purification of linker-modified methylphosphonate oligomers.

The 5'-dimethoxytrityl (trityl) containing oligomers were purified from non-tritylated failure sequences using a Sep-Pak™ C18 cartridge (Millipore/ Waters, Bedford, MA) as follows: The cartridge was washed with acetonitrile, 50% acetonitrile in 100 mM triethylammonium bicarbonate (TEAB, pH 7.5), and 25 mM TEAB. Next, the crude methylphosphonate oligomer was dissolved in a small volume of 1:1 acetonitrile/water and then diluted with 25 mM TEAB to a final concentration of 5% acetonitrile. This solution was then passed through the cartridge. Next, the cartridge was washed with 15-20% acetonitrile in 25 mM TEAB to elute failure sequences from the cartridge. The trityl-on oligomer remaining bound to the cartridge was then detritylated by washing with 25 mM TEAB, 2% trifluoroacetic acid, and 25 mM TEAB, in that order. Finally, the trityl-selected oligomer was eluted from the cartridge with 50% acetonitrile/water and evaporated to dryness under vacuum at room temperature.

The linker-modified methylphosphonate oligomers obtained from the previous step, above, were further purified by reverse-phase HPLC chromatography as follows: A Beckman System Gold HPLC, described in a previous example, was used with a Hamilton PRP-1 column (Reno, NV, 10 µ, 7 mm i.d. x 305 mm long). Buffer A = 50 mM triethylammonium acetate (pH 7); Buffer B = 50% acetonitrile in 50 mM triethylammonium acetate (pH 7). The sample, dissolved in a small volume of 10-50% acetonitrile/water, was loaded onto the column while flowing at 2.5-3 ml/minute with 100% Buffer A. Next, a linear gradient of 0-70% Buffer B was run over 30-50 minutes at a flow rate of 2.5-3 ml/minute. Fractions containing full-length non-nucleotide reagent incorporating methylphosphonate oligomer were evaporated under vacuum and resuspended in 50% acetonitrile/water.

### Example 14

### Preparation of Methylphosphonate Oligomers Incorporating Non-Nucleotide Reagents Which are Targeted to the bcr/abl Region of Chimeric MRNA Associated with CML

The sequence for the bcr/abl junction region was obtained from the K562 cell line as described by G. Grosveld et al. (1986, Molecular and Cellular Biol., 6, pp. 607-616). Methylphosphonate oligomers incorporating non-nucleotide reagents which are complementary to the bcr/abl junction region of this mRNA, were synthesized according to the procedures described in Example 13 herein with the following sequence: 
↓
5'-GGCTTTTGAACTCTGCTT(A)-3'
3'-ACGAUGACCGGCGACUUCCCGAAAACUUGAGACGAAUUUAGG-5'
abl BCR
 The underline indicates sequences originating from the abl gene. One psoralen-conjugated non-nucleotide monomeric unit was incorporated at either ↓ or ↓. At ↓, either a C0, C2, C4, C6 or C8 non-nucleotide monomeric unit was incorporated, to give Oligomers 1, 2, 3, 4 and 5, respectively. At ↓, a C4 non-nucleotide monomeric unit was incorporated to give Oligomer 6. (See Table II). Note: Only oligomer 6 had the 3'-terminal adenine base.

### Example 15

### Preparation of 3' and 5' tandem oligomers

Phosphodiester and methylphosphonate oligomers were prepared with the following sequences by methods described above (See Examples 12 and 13). These oligomers were used to disrupt secondary structure on the RNA strand in the region of the bcr/abl junction:

5'-Tandem oligomer:  5'-GCT-ACT-CCG-CGC-TGA-AG
3'-Tandem oligomer:  5'-AAA-TCC-AGT-GGC-TGA-GTG-3'

The methylphosphonate oligomers were each prepared with a single phosphodiester linkage at the 5'-end to improve their water solubility.

### Example 16

### Reaction of Psoralen-NHS Reagent with Methylphosphonate Oligomers Which Incorporate Non-Nucleotide Monomers

Methylphosphonate oligomers incorporating non-nucleotide monomers (3-5 mg, 99-155 O.D.₂₆₀ units), in 1.5 ml polypropylene microcentrifuge tubes, are dissolved in 100 µl of 1:1 acetonitrile/water. Next, the following reagents are added in order, with vortexing at each addition to avoid precipitation of the oligomers: dimethylsulfoxide (170 µl), water (100 µl) 1 M HEPES buffer, pH 8.0 (50 µl), and 50 mM psoralen-NHS reagent in dimethylsulfoxide (80 µl). Total volume: 500 µl. The mixtures are reacted for 2-4 hours at room temperature with the exclusion of light. Ethanol (1 ml) is then added, and the resulting solutions are chilled at -20°C overnight to precipitate the psoralen labeled oligomer products. The tubes are then spun in a microcentrifuge for 5 minutes and the supernatants are aspirated and discarded. The resulting pellets are resuspended in 500 µl of 1:1 acetonitrile/water and filtered through a 0.22 µ Durapore™ membrane to remove particulates.

The psoralen-labeled methylphosphonate oligomers were purified by reverse-phase HPLC chromatography as follows: A Beckman System Gold analytical HPLC system was used with a Model 126 Solvent module and a Model 167 detector interfaced to an IBM compatible computer and fitted with a Hamilton PRP-1 column (5 µ, 4.1 mm i.d. x 250 mm long). Buffers used were: Buffer A = 50 mM triethylammonium acetate (pH 7); Buffer B = 50% acetonitrile in 50 mM triethylammonium acetate (pH 7). The crude psoralen labeled oligomers were loaded onto the column in five 100 µl portions at two minute intervals with a 500 µl sample loop while the column was flowing at 1.5 ml/minute with 10% Buffer B. NExt, a linear gradient from 10-70% Buffer B was run over 30 minutes at a flow rate of 1.5 ml/min. Fractions were collected at 0.5 minute intervals. Under these conditions, psoralen-labeled oligomers eluted approximately 5 minutes later than the corresponding unlabeled oligomers. Fractions containing psoralen-modified oligomers were pooled and evaporated to dryness under vacuum at room temperature with the exclusion of light. They were then resuspended in a minimal volume of 1:1 acetonitrile/water and quantified by absorbance at 260 nm. Recovered yields ranged from 16% to 55%.

### Example 17

### Cross-Linking of Psoralen-CML Methylphosphonate Oliaomer(3) to a 440-base bcr/abl Transcript

440-base bcr/abl RNA transcript was generated from a pGEM vector clone. This represents a portion of the biological bcr/abl mRNA which contains the bcr/abl junction at approximately the middle of the sequence.

Psoralen-labeled methylphosphonate oligomer 3 which incorporated a non-nucleotide monomeric unit having a C4 linker arm ("oligo 3"), see Table II for sequence, was labeled with ³²P using [γ-³²P]-ATP (3000, Ci/mmol) and T4-polynucleotide kinase as follows: 10 pmol of psoralen methylphosphonate oligomer was dissolved in 10 µl of 50 mM Tris (pH 7.8), 10 mM MgCl₂, 5 mM DTT, 0.1 mM EDTA, 0.1 mM spermidine containing 50 µCi of [γ-³²P]-ATP. T4 polynucleotide kinase (4 units) was added, and the solution was incubated for 90 minutes at room temperature. The radiolabeled product was purified on a Nensorb-20™ column (New England Nuclear/DuPont) according to the manufacturer's instructions.

³²P-labeled psoralen-modified methylphosphonate oligo 3 (0.05 pmol, approximately 20,000 cpm) was added to a 2 ml borosilicate glass autosampler vial containing RNA (1.5 pmols), and tandem phosphate diester oligonucleotides (5 pmol, See Example 15), in 10 µl of 10 mM Tris (pH 7.2), 0.1 mM EDTA, 0.03% potassium sarkosylate. Controls were also prepared with the above reagents along with nonradioactive psoralen-oligo 3 (2 pmols), intended to compete with its radioactive counterpart for the same binding site on the RNA target. The vials were heated at 70°C for 5 minutes, followed by 30 minutes at 35°C and 15 minutes at room temperature. Next, the vials were irradiated at 365 nm on crushed ice with a Model B-100A long wavelength ultraviolet lamp (UVP, Inc., San Gabriel, CA) at a distance of 15 centimeters for 30 minutes. Intensity of irradiation at this distance was approximately 60 µW/100 cm². At the end of the irradiation, 90% formamide containing 0.1% bromphenol blue and 0.1 M tris-borate-EDTA buffer (pH 8.2) was added (5 µl), and the samples were loaded onto a 6% polyacrylamide/7 M urea gel (0.5 mm). The gel was electrophoresed at 900 V for 2 1/2 hours and was then placed between two sheet of Saran Wrap™ and exposed to XAR-5 film (Eastman-Kodak, Rochester, NY) for 15 minutes. Following autoradiography, crosslinking to the RNA target was indicated by the appearance of an upper band. This band was only faintly visible in the controls which contained competing nonradioactive psoralen oligo 3, indicating that the site of crosslinking to the RNA was sequence-specific.

### Example 18

### Comparison of Psoralen Oligos 1, 3 and 5 for Crosslinking to the 440-base bcr/abl RNA Transcript

These oligomers were labeled with ³²P, hybridized, crosslinked to the RNA transcript and analyzed by gel electrophoresis according to the procedure described in Example 17. (See Table II for the sequences of the oligomers.) Prior to autoradiography, however, the gel was transferred to blotting paper and dried in a gel drying apparatus under vacuum at 80°C for two hours. The autoradiograph was then used as a template over the dried gel to facilitate excision of the bands with a scalpel. The excised bands were transferred to 20 ml polypropylene scintillation vials and counted 10 ml of scintillation cocktail (Cytoscint™, ICN Radiopharmaceuticals, Costa Mesa, CA). Extents of crosslinking after 120 minutes of irradiation were as follows:
Psoralen oligo 1 (C0-linker): 5.2%
Psoralen oligo 3 (C4-linker): 14.0%
Psoralen oligo 5 (C8-linker): 4.5%
Based on this observation, it was concluded that the C0- and C8-linkers were too short and too long, respectively, for efficient crosslinking when hybridized to the RNA target.

### Example 19

### Comparison of Psoralen Oligos 2. 3. 4 and 6 for Crosslinking to the 440-base bcr/abl Transcript

These oligomers were labeled with ³²P as described above. (See Table II for sequence.) The radiolabeled oligomers (0.05 pmol, 20-45,000 cpm) were added to 2 ml borosilicate glass autosampler vials containing the RNA target (1 pmol) and tandem methylphosphonate oligomers (5 pmol, above), in 10 µl of 5 mM potassium phosphate, pH 7.4, 0.1 mM EDTA, 0.03% potassium sarkosylate. (We found that the methylphosphonate tandem oligomers promoted greater extents of hybridization and crosslinking of the psoralen-conjugated oligomers to the RNA target under these conditions.) The tubes were heated at 70°C for 5 minutes followed by 30 minutes at 35°C. Next, the tubes were irradiated on ice at 365 nm for 60 minutes as described in Example 17.

Gel analysis, autoradiography and quantification of crosslinking by counting radioactivity in the bands was performed as described in the previous section. The extents of crosslinking to the RNA target were as follows:
Psoralen oligo 2 (C2-linker): 64.9%
Psoralen oligo 3 (C4-linker): 60.7%
Psoralen oligo 4 (C6-linker): 34.5%
Psoralen oligo 6 (C4-linker): 71.6%
This data shows that, for insertion of linkers at the internal position within the oligomer, a C2-linker is slightly preferred over a C4-linker for crosslinking of the attached psoralen moiety to the RNA target strand; the C0-, C6- and C8-linkers are less preferred at this position. Position of a C4-linker at the 3'-end of the oligomer provides a further improvement in crosslinking.

### Example 20

### Reaction of Psoralen-NHS Reagent With an Amine-Modified Methylphosphonate Oligomer

A methylphosphonate oligomer was prepared with a C4-amino linker moiety inserted between two deoxyadenosine bases according to methodology described in a separate patent application. The sequence of this oligomer, which is complementary to the junction region of bcr/abl RNA, is given below: 5'-GGC-TTT-TGA-(L)-ACT-CTG-CTT-3' The bold type bases possess methylphosphonate diester linkages, whereas the 5'-penultimate base is linked by a phosphate diester linkage. The letter "L" designates a non-nucleotide monomeric unit having C4-amino linker described herein.

The following coupling reaction of NHS-psoralen reagent to linker arm of the non-nucleotide monomeric unit (present in the oligomer) was carried out in a 1.5 ml polypropylene microfuge tube. Approximately 3.4 mg (98 OD₂₆₀ units) of the oligomer was dissolved in 100µl of 1:1 acetonitrile/water. Next, the following reagents were added in order, with vortexing at each addition to avoid precipitation of the oligomer:dimethylsulfoxide (170µl), water (100 µl), 1 M HEPES buffer, pH 8.0 (50 µl), and 50 mM psoralen-NHS reagent in dimethylsulfoxide (80 µl). Total volume: 500 µl. The mixture was reacted for 2.5 hours at room temperature in the absence of light. Ethanol (1 ml) was then added, and the resulting solution was chilled at -20°C overnight. The tube was then spun in a microcentrifuge for 5 minutes and the supernatant was aspirated and discarded. The resulting pellet was resuspended in 500 µl of 1:1 acetonitrile/water and filtered through a 0.22 µ Durapore™ membrane to remove particulate material.

HPLC purification of the solution of crude psoralen-oligomer conjugate described above was conducted as follows: A Beckman System Gold analytical HPLC system was used with a Hamilton PRP-1 column (4.1 x 250 mm). Buffers used for elution were: Buffer A - 50 mM triethylammonium acetate (pH 7); Buffer B - 50% acetonitrile in 50 mM triethylammonium acetate (pH 7). The sample was loaded onto the column in five 100 µl portions at two minute intervals with a 500 µl sample loop while the column was flowing at 1.5 ml/min with 10% Buffer B. Next, a linear gradient from 10 - 70% Buffer B was run over 30 minutes. Fractions were collected at 0.5 minute intervals. Under these conditions, unmodified oligomer and psoralen-modified oligomer eluted at 17.9 minutes and 21.7 minutes, respectively. Fractions containing the psoralen-modified oligomer were pooled and evaporated. The overall yield was 16%.

### Example 21

### Cross-Linking of a 440-base bcr/abl RNA Transcript Using a Psoralen Methylphosphonate Oligomer

A 440-base bcr/abl RNA transcript was generated from a pGEM vector clone. This chimeric mRNA is a product of the chimeric gene formed by the translocation of a region of the abl gene into a region of another chromosome containing the bcr gene. This RNA transcript represents a portion of the biological bcr/abl mRNA which contains the bcr/abl junction at approximately the middle of the sequence. In addition, two methylphosphonate oligomers with sequences complementary to adjacent regions on either side of the psoralen methylphosphonate oligomer were synthesized. These tandem oligomers, 17 and 18 bases in length respectively, were used to disrupt secondary structure on the RNA strand in the region of the bcr/abl junction.

The psoralen methylphosphonate oligomer conjugate was labeled with ³²P using [γ-³²P]-ATP (3000 Ci/mmol) and T4 polynucleotide kinase as follows: 10 pmol of psoralen methylphosphonate oligonucleotide was dissolved in 10 µl of 50 mM Tris (pH 7.8), 10 mM MgCl₂, 5 mM DTT, 0.1 mM EDTA, 0.1 mM spermidine containing 50 µCi of [γ-³²P]-ATP. T4 polynucleotide kinase (4 units) was added, and the solution was incubated for 90 minutes at room temperature. The radiolabeled product was purified on a Nensorb-20™ column (New England Nuclear/DuPont) according to the manufacturer's instructions.

In an example of a cross-linking experiment, ³²P-labeled psoralen oligomer conjugate (50,000 CPM) was added to a 2 ml borosilicate glass autosampler vial along with RNA (1 pmol) and the tandem methylphosphonate oligomers (5 pmol) in 10 µl of buffer consisting of 5 mM potassium phosphate (pH 7.4), 0.1 mM EDTA and 0.03% potassium sarkosyl. The vial was heated at 70°C for three minutes and then incubated at 30°C for 30 minutes. Next, the vials were placed on crushed ice and irradiated at 365 nm with a Model B-100A long wavelength ultraviolet lamp (UVP, Inc., San Gabriel, CA) at a distance of 15 centimeters. Intensity of irradiation averaged 60 µW/100 cm². Under these conditions, cross-linking was 80-90% complete after 30 minutes. Next, 90% formamide containing 0.1% bromphenol blue was added (5 µl) and the sample was loaded onto a 6% polyacrylamide gel containing 7 M urea (0.5 mm). The gel was electrophoresed at 900 volts for 2 hours and then transferred to blotting paper and dried. Autoradiography was done using XAR-5 film (Eastman-Kodak, Inc.) for 5-12 hours. Bands were quantified by cutting them out of the gel and counting in a scintillation counter in the presence of Cytoscint™ scintillation cocktail (ICN Radiopharmaceuticals, Inc., Costa Mesa, CA). The upper band corresponded to psoralen oligonucleotide cross-linked to the RNA target.

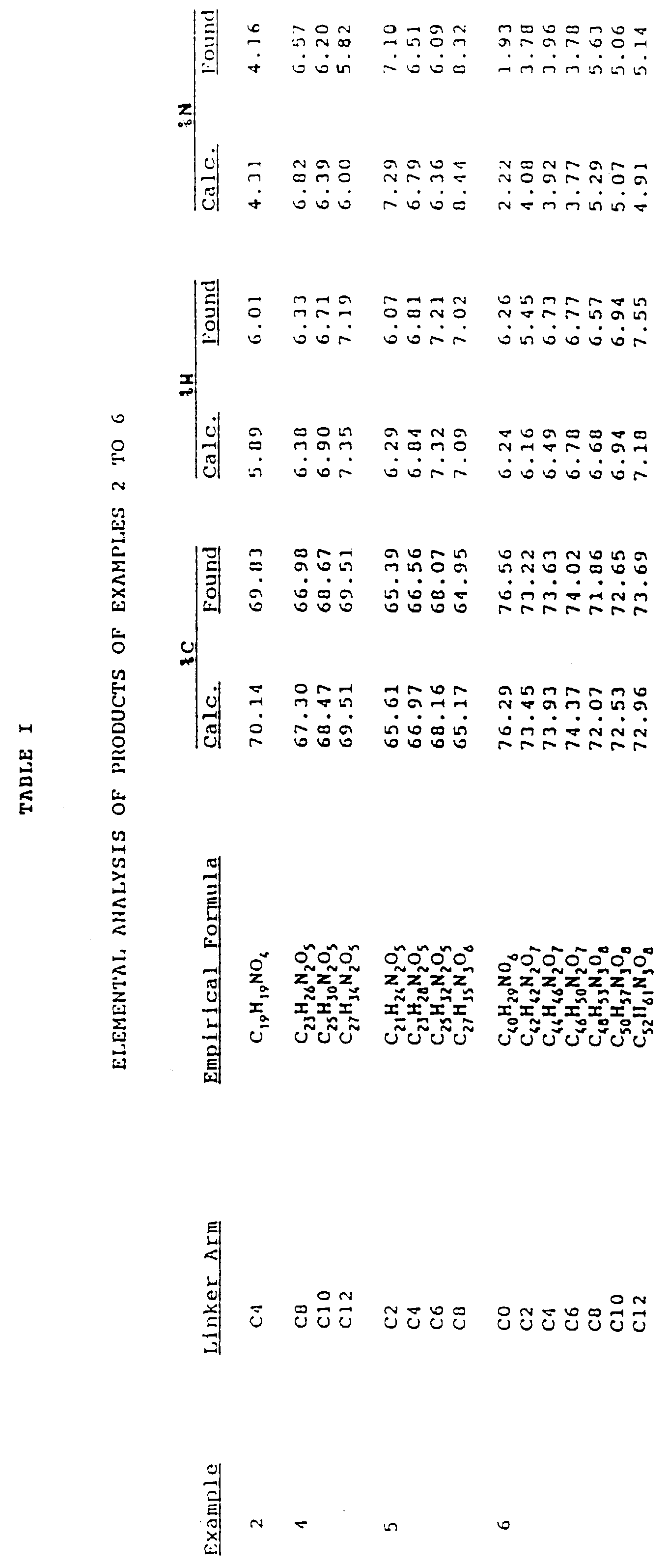

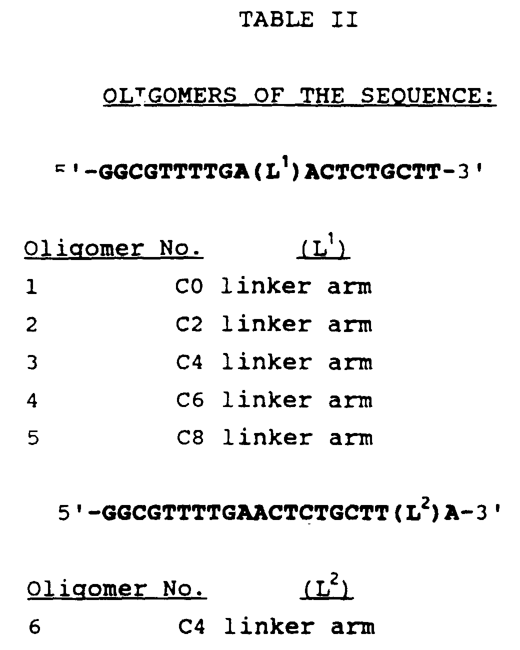

## Claims

1. A reagent for attaching a psoralen moiety (-Ps) to an oligomer which comprises a compound of the formula: 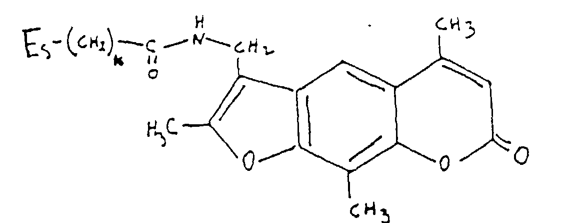 wherein k is an integer from 0 to 12, and Es is a moiety capable of coupling with a nucleophilic moiety.

2. A reagent according to claim 1 wherein Es comprises an activated ester with a leaving group which is readily displaced by a nucleophilic moiety.

3. A reagent according to claim 1 wherein Es is a N-hydroxysuccinimide activated ester.

4. A reagent according to claim 3 wherein k is 2.

5. A reagent according to claim 4 wherein Es comprises:

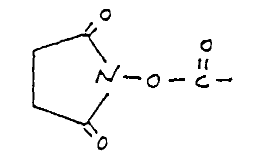

6. An oligomer which is complementary to bcr/abl of a chimeric mRNA transcript of the Philadelphia chromosome and which incorporates at least one non-nucleotide monomeric unit having a psoralen moiety (-Ps) conjugated thereto,
wherein Ps has the formula: 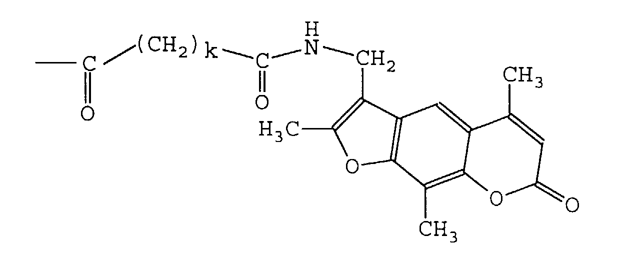 wherein k is an integer from 0 to 12.

7. An oligomer according to claim 6 which comprises an alkyl- or aryl-phosphonate oligomer.

8. An oligomer according to claim 6 wherein said non-nucleotide monomeric unit comprises: 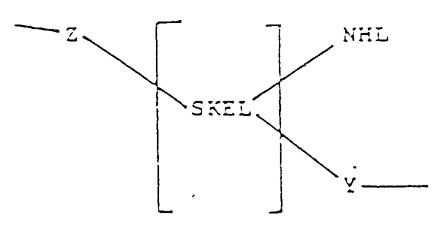 wherein SKEL comprises a chirally pure non-nucleotide skeleton of 1 to 20 carbon atoms, wherein -NHL, Y and Z are covalently linked to a carbon atom of SKEL, L is a ligand, Y is -CH₂-, -O-, -S- or -NH-; and Z is -O-, -S- or -NH-.

9. An oligomer according to claim 8 wherein SKEL comprises a backbone of 1 to 10 carbon atoms between Y and Z.

10. An oligomer according to claim 9 wherein L is selected from -Ps, 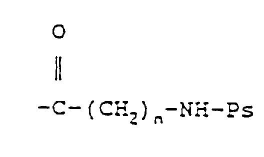 and 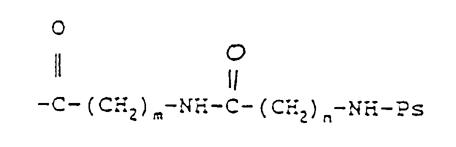 wherein n and m are independently integers from 1 to 15 and PS is a psoralen moiety as defined in claim 6.

11. An oligomer according to claim 10 which comprises an alkyl- or aryl-phosphonate oligomer.

12. An oligomer according to claim 11 having nucleotide monomeric units which comprise an 2'-O-alkyl ribosyl moiety.

13. An oligomer according to Claim 6 wherein said non-nucleotide monomeric unit comprises: 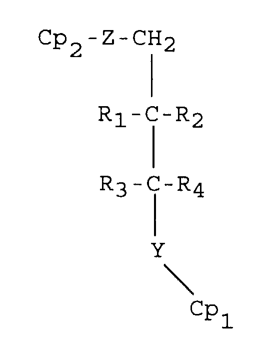 wherein one of R₁ and R₂ is hydrogen and the other is -NH- L wherein L is a linker arm conjugated to a psoralen moiety as defined in claim 6; one of R₃ and R₄ is hydrogen and the other is lower alkyl of 1 to 10 carbon atoms; wherein -Y-Cp₁ is a first coupling group wherein Y is -CH₂-, sulphur, amine, or oxygen and Cp₁ is selected from 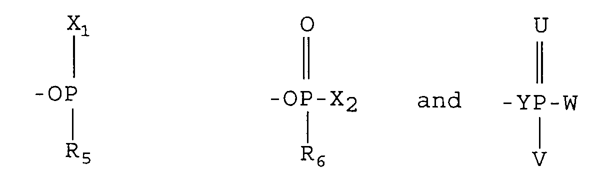 wherein X₁ is halogen or substituted amino; X₂ is halogen, amino, or substituted amino, or O; R₅ is alkyl, substituted alkoxy or substituted aryloxy; and R₆ is alkyl, substituted alkoxy or substituted aryloxy or hydrogen when X₂ is O; U is oxygen or sulfur, W is alkyl, aryl, alkoxy, alkylthio, aryloxy, arylthio, O, S, amino or substituted amino, and V is alkoxy, alkylthio amino or substituted amino; and -Z-Cp₂ is a blocked second coupling group, wherein Cp₂ is a blocking group cleavable under deblocking conditions to recover the second coupling group -ZH wherein Z is -O-, -NH- or -S-.

14. An oligomer according to claim 13 wherein L comprises -Ps or a linker arm conjugated to a psoralen moiety selected from: 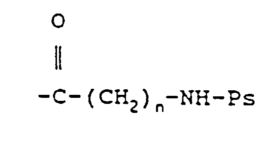 and 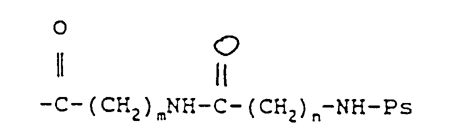 wherein n and m are independently an integer from 1 to 15 and Ps is a psoralen moiety as defined in claim 6.

15. An oligomer according to claim 14 wherein L is

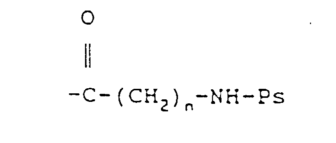

16. An oligomer according to claim 15 wherein n is 1 or 3.

17. An oligomer according to claim 6 wherein said oligomer is capable of hybridizing with a portion of the region coding for P210^{bcr/abl}.

18. An oligomer which incorporates at least one non-nucleotide monomeric unit having a psoralen moiety as defined in claim 6 conjugated thereto and which is capable of hybridizing to bcr/abl in chimeric mRNA.

19. An oligomer according to claim 18 which is capable of interfering with expression of P210.

20. The use of an oligomer, which is complementary to a portion of the bcr/abl region and effective to prevent expression of P210^{bcr/abl}, in the manufacture of a medicament effective against chronic myelogenous leukaemia or isolated cells thereof.

21. A use according to Claim 20 wherein said cells comprise bone marrow cells.

22. A use according to Claim 20 wherein said oligomer comprises at least one non-nucleotide monomeric unit.

23. A use according to Claim 20 wherein psoralen is covalently attached to a non-nucleotide monomeric unit of said oligomer.

24. A use according to Claim 23, wherein said oligomer comprises a methylphosphonate oligomer.

25. A use according to Claim 23, wherein said oligomer comprises from 6 to 25 nucleotide monomeric units.

26. A use according to Claim 25 wherein said oligomer comprises from 1 to 5 independently selected non-nucleotide monomeric units.

27. A use according to Claim 26, wherein a second tandem oligomer is included which is complementary to a sequence on bcr/abl in RNA 5' or 3' to the sequence complementary to said oligomer.

28. A use according to Claim 27, wherein said tandem oligomer comprises an alkyl- or aryl-phosphonate oligomer.

29. A use according to Claim 27, wherein said tandem oligomer comprises a methylphosphonate oligomer.

30. The use of an oligomer, which is complementary to a nucleic acid coding for a protein of the abl gene or mRNA, effective to decrease expression of tyrosine kinase in the manufacture of a medicament effective against chronic myelogenous leukaemia or isolated cells thereof.

31. A use according to Claim 30, wherein said oligomer comprises at least one psoralen-conjugated non-nucleotide monomeric unit.

32. A use according to Claim 31 wherein said oligomer comprises a methylphosphonate oligomer.

33. An oligomer which is complementary to a nucleic acid sequence which is a product of a genetic translocation wherein said oligomer comprises at least one non-nucleotide monomeric unit having a psoralen moiety as defined in claim 6 conjugated thereto.

34. An oligomer according to Claim 33 wherein said non-nucleotide monomeric unit comprises: 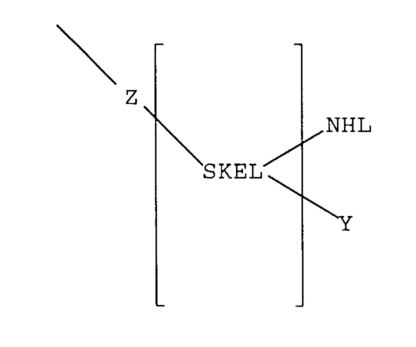 wherein SKEL comprises a chirally pure non-nucleotide skeleton of 1 to 20 carbon atoms, wherein - NHL, Y, and Z are covalently linked to a carbon atom of SKEL, L is a ligand, Y is -CH₂-, -O-, -S- or -NH-; and Z is -O-,-S- or -NH-.

35. An oligomer according to Claim 34, wherein SKEL comprises a backbone of 1 to 10 carbon atoms between Y and Z.

36. An oligomer according to Claim 35, wherein L is selected from -Ps, 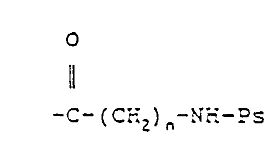 and 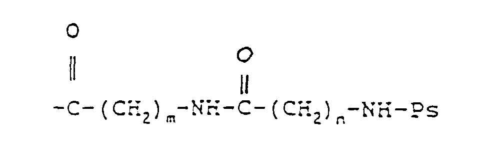 wherein n and m are independently integers from 1 to 15 and Ps is a psoralen moiety as defined in claim 6.

37. An oligomer according to Claim 36, which comprises an alkyl- or aryl-phosphonate oligomer.

38. An oligomer according to Claim 36, which comprises a methylphosphonate oligomer.

39. An oligomer according to Claim 38, which comprises nucleoside monomeric units having a 2'-O-methyl-ribosyl moiety.

40. An oligomer according to Claim 38, which comprises from 6 to 31 monomeric units.

41. An oligomer according to Claim 40 which comprises from 1 to 5 independently selected non-nucleotide monomeric units.

42. An oligomer according to Claim 36, which comprises from 6 to 31 monomeric units.

43. An oligomer according to Claim 42 which comprises from 1 to 5 independently selected non-nucleotide units.

44. An oligomer according to Claim 33, wherein k is 2 to 6.

45. An oligomer according to Claim 44, wherein k is 2.

46. An oligomer according to Claim 33, wherein said non-nucleotide monomeric unit comprises: 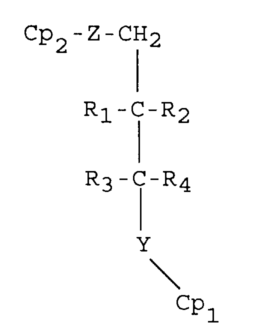 wherein one of R₁ and R₂ is hydrogen and the other is -NH- L wherein L is a linker arm conjugated to a psoralen moiety as defined in claim 6; one of R₃ and R₄ is hydrogen and the other is lower alkyl of 1 to 10 carbon atoms; wherein -Y-Cp₁ is a first coupling group wherein Y is -CH₂-, sulfur, amine, or oxygen and Cp₁ is selected from 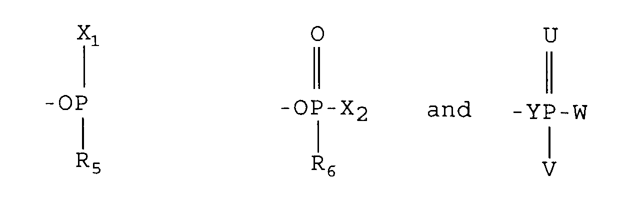 wherein X₁ is halogen or substituted amino; X₂ is halogen, amino, or substituted amino, or O; R₅ is alkyl, substituted alkoxy or substituted aryloxy; and R₆ is alkyl, substituted alkoxy or substituted aryloxy or hydrogen when X₂ is O; U is oxygen or sulfur, W is alkyl, aryl, alkoxy, alkylthio, aryloxy, arylthio, O,S, amino or substituted amino: and V is alkoxy, alkylthio, amino or substituted amino; and -Z-Cp₂ is a blocked second coupling group, wherein Cp₂ is a blocking group cleavable under deblocking conditions to recover the second coupling group -ZH wherein Z is -O-, -NH- or -S-.

47. An oligomer according to Claim 46, wherein L is selected from -Ps, 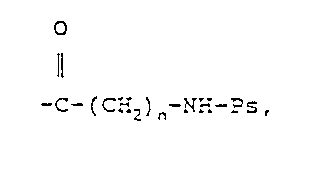 and 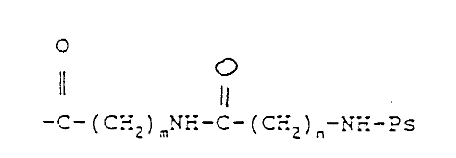 wherein n and m are independently integers from 1 to 15 and Ps is a psoralen moiety as defined in claim 6.

48. An oligomer according to Claim 47, which comprises a methylphosphonate oligomer.

49. An oligomer according to Claim 48, wherein L is 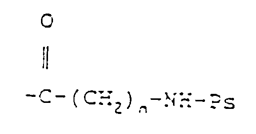 wherein n is an integer from 1 to 5.

50. An oligomer according to Claim 49 which comprises from 6 to 31 monomeric units.

51. An oligomer according to Claim 50, which comprises from 1 to 5 independently selected non-nucleotide monomeric units.

52. An oligomer according to Claim 51, wherein said non-nucleotide monomeric units are chirally pure.

## Patentansprüche

1. Reagens zur Anheftung eines Psoralen-Teils (-Ps) an ein Oligomer, das eine Verbindung der folgenden Formel umfaßt: 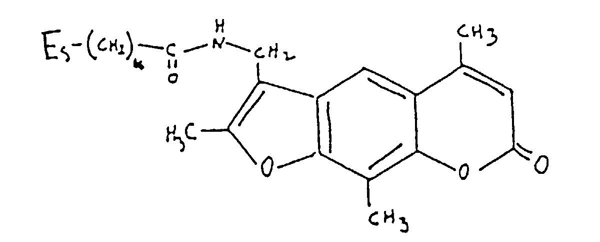 worin k eine ganze Zahl von 0 bis 12 ist, und Es eine Gruppe ist, die fähig ist, sich mit einer nukleophilen Gruppe zu verbinden.

2. Reagens nach Anspruch 1, worin Es einen aktivierten Ester mit einer Abgangsgruppe, die leicht durch eine nukleophile Gruppe ersetzt wird, umfaßt.

3. Reagens nach Anspruch 1, worin Es ein durch N-Hydroxysuccinimid aktivierter Ester ist.

4. Reagens nach Anspruch 3, worin k 2 ist.

5. Reagens nach Anspruch 4, worin Es 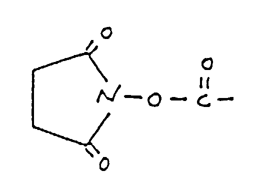 umfaßt.

6. Oligomer, das zu bcr/abl eines chimären mRNA-Transkripts des Piladelphia-Chromosoms komplementär ist und das mindestens eine monomere Nicht-Nukleotid-Einheit, die einen daran gebundenen Psoralen-Teil (-Ps) hat, beinhaltet, wobei Ps die folgende Formel hat 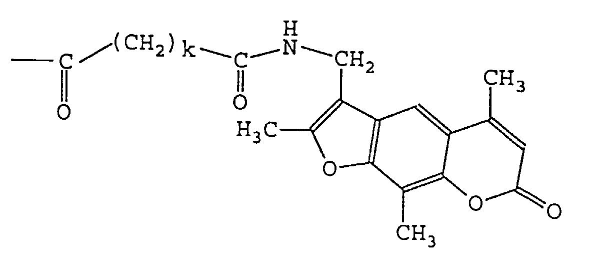 worin k eine ganze Zahl von 0 bis 12 ist.

7. Oligomer nach Anspruch 6, das ein Alkyl- oder Arylphosphonat-Oligomer umfaßt.

8. Oligomer nach Anspruch 6, wobei die monomeren Nicht-Nukleotid-Einheit 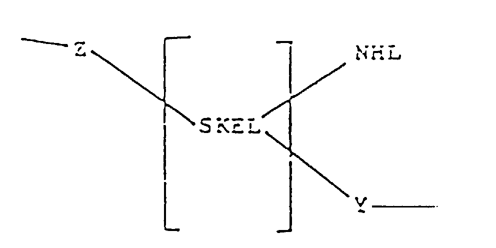 umfaßt, worin SKEL ein chiral reines Nicht-Nukleotid-Gerüst mit 1 bis 20 Kohlenstoffatomen umfaßt; NHL, Y und Z kovalent an ein Kohlenstoffatom von SKEL gebunden sind; L ein Ligand ist; Y -CH₂-, -O-, -S- oder -NH- ist; und Z -O-, -S- oder -NH- ist.

9. Oligomer nach Anspruch 8, worin SKEL zwischen Y und Z eine Hauptkette aus 1 bis 10 Kohlenstoffatomen enthält.

10. Oligomer nach Anspruch 9, worin L unter -Ps, 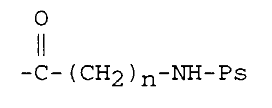 und 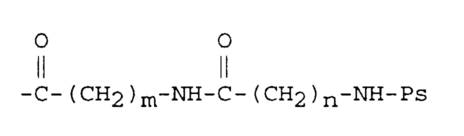 in denen n und m unabhängig voneinander ganze Zahlen von 1 bis 15 sind, und PS ein Psoralen-Teil ist, wie er in Anspruch 6 definiert ist, ausgewählt ist.

11. Oligomer nach Anspruch 10, das ein Alkyl- oder Arylphosphonat-Oligomer umfaßt.

12. Oligomer nach Anspruch 11, das monomere Nukleotid-Einheiten enthält, welche eine 2'-O-Alkylribosyl-Gruppe umfassen.

13. Oligomer nach Anspruch 6, worin die monomere Nicht-Nukleotid-Einheit 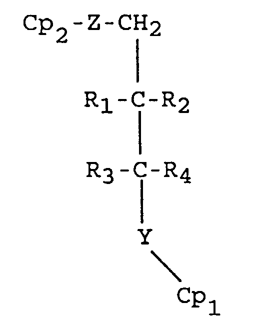 umfaßt, worin einer der Reste R₁ und R₂ Wasserstoff und der andere -NH-L, worin L ein Linkerarm ist, der an einen Psoralen-Teil, wie er in Anspruch 6 definiert ist, gebunden ist, darstellt; einer der Reste R₃ und R₄ Wasserstoff und der andere ein Niederalkyl mit 1 bis 10 Kohlenstoffatomen ist; worin -Y-Cp₁ eine erste Verknüpfungsgruppe ist, in der Y -CH₂-, Schwefel, Amin oder Sauerstoff ist, und Cp₁ aus 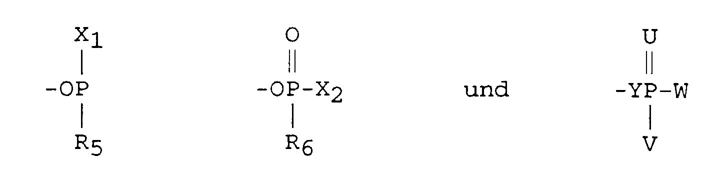 worin X₁ Halogen oder substituiertes Amino ist; X₂ Halogen, Amino oder substituiertes Amino oder O ist; R₅ Alkyl, substituiertes Alkoxy oder substituiertes Aryloxy ist; und R₆ Alkyl, substituiertes Alkoxy oder substituiertes Aryloxy oder Wasserstoff, wenn X₂ O ist, ist; U Sauerstoff oder Schwefel ist; W Alkyl, Aryl, Alkoxy, Alkylthio, Aryloxy, Arylthio, O, S, Amino oder substituiertes Amino ist; und V Alkoxy, Alkylthio, Amino oder substituiertes Amino ist; ausgewählt ist; und -Z-Cp₂ eine blockierte zweite Verknüpfungsgruppe ist, worin Cp₂ eine Blockierungsgruppe ist, die unter Entblockungsbedingungen spaltbar ist, wobei die zweite Verknüpfungsgruppe -ZH, in der Z -O-, -NH- oder -S- ist, wiederhergestellt wird.

14. Oligomer nach Anspruch 13, in dem L -Ps oder einen Linkerarm, der an einen Psoralen-Teil gebunden ist, ausgewählt aus: 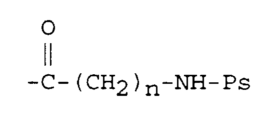 und 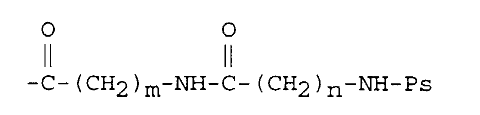 worin n und m unabhängig voneinander eine ganze Zahl zwischen 1 und 15 sind, und Ps ein Psoralen-Teil, wie er in Anspruch 6 definiert ist, darstellt, umfaßt.

15. Oligomer nach Anspruch 14, worin L 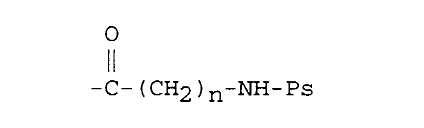 ist.

16. Oligomer nach Anspruch 15, worin n 1 oder 3 ist.

17. Oligomer nach Anspruch 6, wobei das Oligomer fähig ist, mit einem Teil der Region, die P210^{bcr/abl} kodiert, zu hybridisieren.

18. Oligomer, das mindestens eine monomere Nicht-Nukleotid-Einheit, die einen daran gebundenen Psoralen-Teil, wie er in Anspruch 6 definiert ist, hat und die fähig ist, mit bcr/abl in chimerer mRNA zu hybridisieren, einschließt.

19. Oligomer nach Anspruch 8, das fähig ist, in eine Expression von P210 einzugreifen.

20. Verwendung eines Oligomers, das zu einem Teil der bcr/abl-Region komplementär ist und zur Verhinderung einer Expression von P210^{bcr/abl} wirksam ist, bei der Herstellung eines Arzneimittels, das gegen konische myeloische Leukämie oder isolierte Zellen derselben wirksam ist.

21. Verwendung nach Anspruch 20, wobei die Zellen Knochenmarkzellen umfassen.

22. Verwendung nach Anspruch 20, wobei das Oligomer mindestens eine monomere Nicht-Nukleotid-Einheit umfaßt.

23. Verwendung nach Anspruch 20, wobei Psoralen kovalent an eine monomere Nicht-Nukleotid-Einheit des Oligomers gebunden ist.

24. Verwendung nach Anspruch 23, wobei das Oligomer ein Methylphosphonatoligomer umfaßt.

25. Verwendung nach Anspruch 23, wobei das Oligomer 6 bis 25 monomere Nukleotid-Einheiten umfaßt.

26. Verwendung nach Anspruch 25, wobei das Oligomer zwischen 1 und 5 unabhängig voneinander ausgewählte monomere Nicht-Nukleotid-Einheiten umfaßt.

27. Verwendung nach Anspruch 26, wobei ein zweites Tandem-Oligomer enthalten ist, welches zu einer Sequenz von bcr/abl in 5'-RNA oder 3'-RNA zu der Sequenz komplementär zu dem Oligomer komplementär ist.

28. Verwendung nach Anspruch 27, wobei das Tandem-Oligomer ein Alkyl- oder Arylphosphonat-Oligomer umfaßt.

29. Verwendung nach Anspruch 27, wobei das Tanden-Oligomer ein Methylphosphonat-Oligomer umfaßt.

30. Verwendung eines Oligomers, das zu einer Nukleinsäure, die für ein Protein des abl-Gens kodiert, oder mRNA komplementär ist, das zur Verringerung der Expression von Tyrosinkinase wirksam ist, bei der Herstellung eines Arzneimittels, das gegen chronische myeloische Leukämie oder isolierte Zellen derselben wirksam ist.

31. Verwendung nach Anspruch 30, wobei das Oligomer mindestens eine Psoralen-gebundene monomere Nicht-Nukleotid-Einheit umfaßt.

32. Verwendung nach Anspruch 31, wobei das Oligomer ein Methylphosphonat-Oligomer umfaßt.

33. Oligomer, das zu einer Nukleinsäuresequenz, welche ein Produkt einer genetischen Translokation ist, komplementär ist, wobei das Oligomer mindestens eine monomere Nicht-Nukleotid-Einheit, die einen Psoralen-Teil, wie er in Anspruch 6 definiert ist, gebunden hat, umfaßt.

34. Oligomer nach Anspruch 33, wobei die monomere Nicht-Nukleotid-Einheit 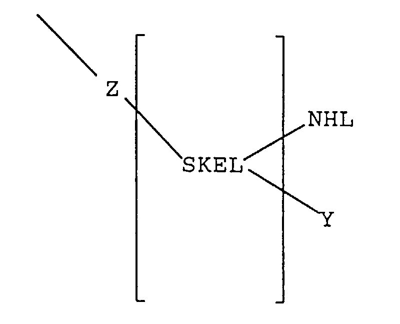 enthält, worin SKEL ein chiral reines Nicht-Nukleotid-Gerüst mit 1 bis 20 Kohlenstoffatomen umfaßt, NHL, Y und Z kovalent an ein Kohlenstoffatom von SKEL gebunden sind; L ein Ligand ist; Y -CH₂-, -O-, -S- oder -NH- ist; und Z -O-, -S- oder -NH- ist.

35. Oligomer nach Anspruch 34, wobei SKEL zwischen Y und Z eine Hauptkette aus 1 bis 10 Kohlenstoffatomen umfaßt.

36. Oligomer nach Anspruch 35, wobei L unter -Ps 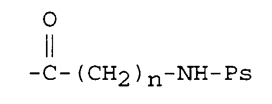 und 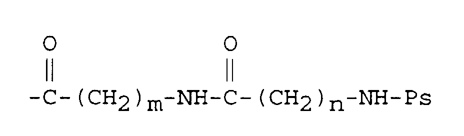 in denen n und m unabhängig voneinander ganze Zahlen zwischen 1 und 15 sind, und Ps ein Psoralen-Teil, wie er in Anspruch 6 definiert ist, ist, ausgewählt ist.

37. Oligomer nach Anspruch 36, das ein Alkyl- oder Arylphosphonat-Oligomer umfaßt.

38. Oligomer nach Anspruch 36, das ein Methylphosphonat-Oligomer umfaßt.

39. Oligomer nach Anspruch 38, das monomere Nukleosid-Einheiten mit einer 2'-O-Methyl-ribosyl-Gruppe umfaßt.

40. Oligomer nach Anspruch 38, das 6 bis 31 monomere Einheiten umfaßt.

41. Oligomer nach Anspruch 40, das 1 bis 5 unabhängig voneinander ausgewählte monomere Nicht-Nukleotid-Einheiten umfaßt.

42. Oligomer nach Anspruch 36, das zwischen 6 und 31 monomere Einheiten umfaßt.

43. Oligomer nach Anspruch 42, das zwischen 1 und 5 unabhängig voneinander ausgewählte Nicht-Nukleotid-Einheiten umfaßt.

44. Oligomer nach Anspruch 33, worin k 2 bis 6 ist.

45. Oligomer nach Anspruch 44, worin k 2 ist.

46. Oligomer nach Anspruch 33, worin die monomere Nicht-Nukleotid-Einheit 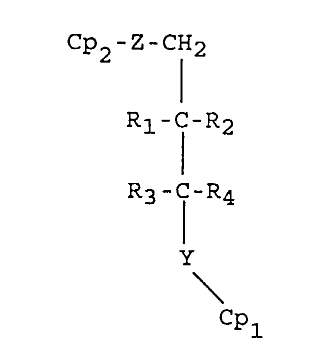 worin einer der Reste R₁ und R₂ Wasserstoff und der andere -NH-L, worin L ein Linkerarm ist, welcher an eine Psoralen-Teil, wie er in Anspruch 6 definiert ist, gebunden ist, ist; einer der Reste R₃ und R₄ Wasserstoff und der andere ein Niederalkyl mit 1 bis 10 Kohlenstoffatomen ist; -Y-Cp₁ eine erste Verknüpfungsgruppe ist, worin Y -CH₂-, Schwefel, Amin oder Sauerstoff ist, und Cp₁ unter 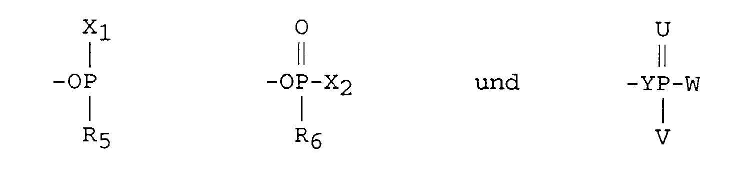 in denen X₁ Halogen oder substituiertes Amino ist; X₂ Halogen, Amino oder substituiertes Amino oder O ist; R₅ Alkyl, substituiertes Alkoxy oder substituiertes Aryloxy ist; und R₆ Alkyl, substituiertes Alkoxy oder substituiertes Aryloxy oder Wasserstoff, wenn X₂ O ist, ist; U Sauerstoff oder Schwefel ist; W Alkyl, Aryl, Alkoxy, Alkylthio, Aryloxy, Arylthio, O, S, Amino oder substituiertes Amino ist; und V Alkoxy, Alkylthio, Amino oder substituiertes Amino ist; ausgewählt ist, und -Z-Cp₂ eine blockierte zweite Verknüpfungsgruppe ist, worin Cp₂ eine Blockierungsgruppe ist, die unter Entblockierungsbedingungen unter Wiederherstellung der zweiten Verknüpfungsgruppe -ZH, worin Z -O-, -NH- oder -S- ist, spaltbar ist, umfaßt.

47. Oligomer nach Anspruch 46, worin L unter -Ps, 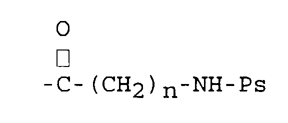 und 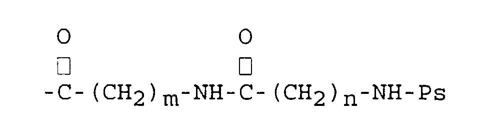 worin n und m unabhängig voneinander ganze Zahlen zwischen 1 und 15 sind, und Ps ein Psoralen-Teil, wie er in Anspruch 6 definiert ist, ist, ausgewählt ist.

48. Ein Oligomer nach Anspruch 47, das ein Methylphosphonat-Oligomer umfaßt.

49. Oligomer nach Anspruch 48, worin L 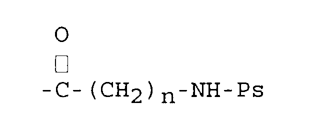 in der n eine ganze Zahl von 1 bis 5 ist, ist.

50. Oligomer nach Anspruch 49, das zwischen 6 und 31 monomere Einheiten umfaßt.

51. Oligomer nach Anspruch 50, das zwischen 1 und 5 unabhängig voneinander ausgewählte monomere Nicht-Nukleotid-Einheiten umfaßt.

52. Oligomer nach Anspruch 51, worin die monomeren Nicht-Nukleotid-Einheiten chiral rein sind.

## Revendications

1. Réactif pour attacher un fragment psoralène (-Ps) à un oligomère qui comprend un composé de formule : 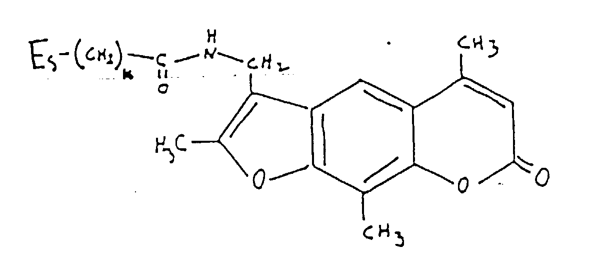 dans laquelle k représente un nombre entier de 0 à 12, et Es représente un fragment capable de se coupler avec un fragment nucléophile.

2. Réactif selon la revendication 1, dans lequel Es comprend un ester activé avec un groupe qui part qui est facilement déplacé par un fragment nucléophile.

3. Réactif selon la revendication 1, dans lequel Es représente un groupe ester activé de N-hydroxysuccinimide.

4. Réactif selon la revendication 3, dans lequel k est égal à 2.

5. Réactif selon la revendication 4, dans lequel Es comprend :

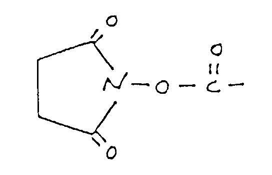

6. Oligomère qui est complémentaire au bcr/abl d'un transcripteur d'ARN messager chimère du chromosome Philadelphie et qui comprend au moins un motif monomère non-nucléotide comportant un fragment psoralène (-Ps) conjugué à celui-ci, dans lequel Ps a pour formule : 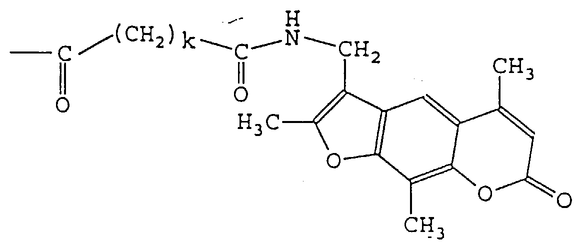 dans laquelle k représente un nombre entier de 0 à 12.

7. Oligomère selon la revendication 6 qui comprend un oligomère d'alkyl- ou aryl-phosphonate.

8. Oligomère selon la revendication 6, dans lequel ledit motif monomère non-nucléotide comprend : 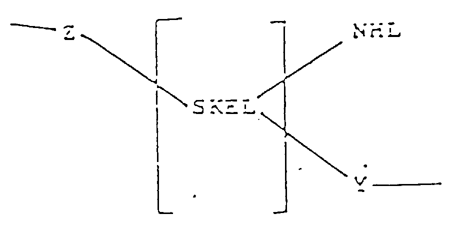 où SKEL comprend un squelette non-nucléotide chiralement pur, comportant de 1 à 20 atomes de carbone, où -NHL, Y et Z sont liés par covalence à un atome de carbone de SKEL, L représente un ligand, Y représente -CH₂-, -O-, -S- ou -NH-; et Z représente -O-, -S- ou -NH-.

9. Oligomère selon la revendication 8, dans lequel SKEL comprend un tronc comportant de 1 à 10 atomes de carbone entre Y et Z.

10. Oligomère selon la revendication 9, dans lequel L est choisi parmi -Ps, 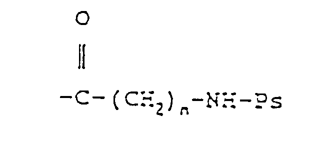 et 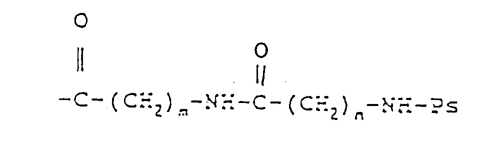 où n et m représentent indépendamment des nombres entiers de 1 à 15, Ps représente un fragment psoralène selon la revendication 6.

11. Oligomère selon la revendication 10 qui comprend un oligomère d'alkyl- ou aryl-phosphonate.

12. Oligomère selon la revendication 11, comportant des motifs monomères nucléotides qui comprennent un fragment 2'-O-alkylribosyle.

13. Oligomère selon la revendication 6, dans lequel le motif monomère non-nucléotide comprend : 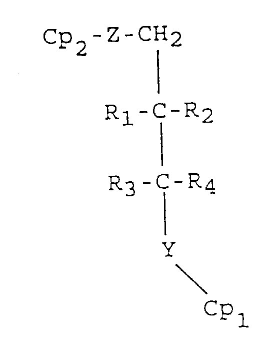 où l'un des symboles R₁ et R₂ représente un atome d'hydrogène et l'autre représente -NH-L où L représente un bras de liaison conjugué à un fragment psoralène selon la revendication 6; un des symboles R₃ et R₄ représente un atome d'hydrogène et l'autre représente un groupe alkyle inférieur comportant de 1 à 10 atomes de carbone; où -Y-Cp₁ représente un premier groupe de couplage où Y représente -CH₂-, un atome de soufre, un groupe amino ou un atome d'oxygène et Cp₁ est choisi parmi : 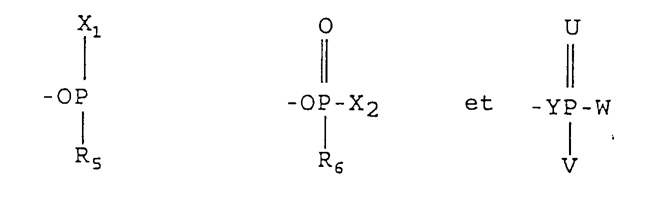 où X₁ représente un atome d'halogène ou un groupe amino substitué; X₂ représente un atome d'halogène, un groupe amino, ou un groupe amino substitué, ou O; R₅ représente un groupe alkyle, un groupe alcoxy substitué ou un groupe aryloxy substitué; et R₆ représente un groupe alkyle, un groupe alcoxy substitué ou un groupe aryloxy substitué ou un atome d'hydrogène quand X₂ représente O; U représente un atome d'oxygène ou de soufre, W représente un groupe alkyle, un groupe aryle, un groupe alcoxy, un groupe alkylthio un groupe aryloxy, un groupe arylthio, O, S, un groupe amino ou amino substitué, et V représente un groupe alcoxy, un groupe alkylthio, un groupe amino ou amino substitué; et Z-Cp₂ représente un second groupe de couplage bloqué, où Cp₂ représente un groupe de blocage clivable dans des conditions de déblocage pour récupérer le second groupe de couplage -ZH où Z représente -0-, -NH- ou -S-.

14. Oligomère selon la revendication 13, dans lequel L comprend -Ps ou un bras de liaison conjugué à un fragment psoralène choisi parmi : 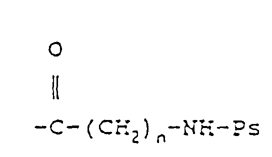 et 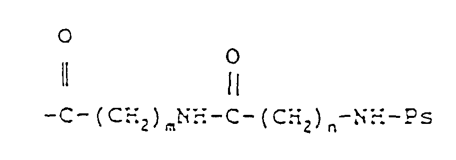 où n et m représentent indépendamment un nombre entier de 1 à 15 et Ps représente un fragment psoralène selon la revendication 6

15. Oligomère selon la revendication 14, dans lequel L représente

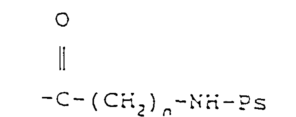

16. Oligomère selon la revendication 15, dans lequel n est égal à 1 ou à 3.

17. Oligomère selon la revendication 6, dans lequel ledit oligomère est capable de former un hybride avec une partie de la région codant pour P210^{bcr/abl}.

18. Oligomère qui comprend au moins un motif monomère non-nucléotide ayant un fragment psoralène selon la revendication 6, conjugué à celui-ci et qui est capable de former un hybride avec bcr/abl dans l'ARN messager chimère.

19. Oligomère selon la revendication 18 qui est capable d'interférer avec l'expression de P210.

20. Utilisation d'un oligomère, qui est complémentaire à une partie de la région bcr/abl et efficace pour empêcher l'expression de P210^{bcr/abl}, dans la fabrication d'un médicament efficace contre la leucémie myélogène chronique ou des cellules isolées de celle-ci.

21. Utilisation selon la revendication 20, dans laquelle lesdites cellules comprennent des cellules de moëlle osseuse.

22. Utilisation selon la revendication 20, dans laquelle ledit oligomère comprend au moins un motif monomère non-nucléotide.

23. Utilisation selon la revendication 20, dans lequel le psoralène est attaché par covalence à un motif monomère non-nucléotide dudit oligomère.

24. Utilisation selon la revendication 23, dans laquelle ledit oligomère comprend un oligomère de méthylphosphonate.

25. Utilisation selon la revendication 23, dans laquelle ledit oligomère comprend de 6 à 25 motifs monomères nucléotides.

26. Utilisation selon la revendication 25, dans laquelle ledit oligomère comprend de 1 à 5 motifs monomères non-nucléotides choisis indépendamment.

27. Utilisation selon la revendication 26, dans laquelle un second oligomère en tandem est inclus qui est complémentaire à une séquence sur bcr/abl dans l'ARN 5' ou 3' à la séquence complémentaire audit oligomère.

28. Utilisation selon la revendication 27, dans laquelle ledit oligomère en tandem comprend un oligomère d'alkyl- ou aryl-phosphonate.

29. Utilisation selon la revendication 27, dans lequel ledit oligomère en tandem comprend un oligomère de méthylphosphonate.

30. Utilisation d'un oligomère, qui est complémentaire à un acide nucléique codant pour une protéine du gène abl ou d'ARN messager, efficace pour diminuer l'expression de la tyrosine kinase dans la fabrication d'un médicament- efficace envers la leucémie myélogène chronique ou des cellules isolées de celle-ci.

31. Utilisation selon la revendication 30, dans laquelle ledit oligomère comprend au moins un motif monomère non-nucléotide conjugué au psoralène.

32. Utilisation selon la revendication 31, dans laquelle ledit oligomère comprend un oligomère de méthylphosphonate.

33. Oligomère qui est complémentaire à une séquence d'acide nucléique qui est un produit d'une translocation génétique où ledit oligomère comprend au moins un motif monomère non-nucléotide comportant un fragment psoralène selon la revendication 6 conjugué à celui-ci.

34. Oligomère selon la revendication 33, dans lequel ledit motif monomère non-nucléotide comprend : 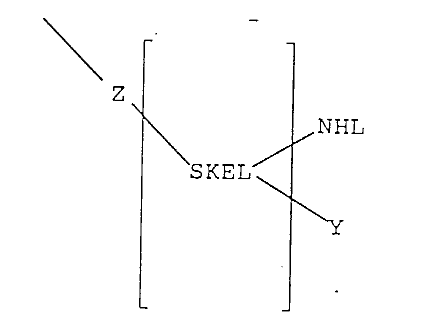 où SKEL comprend un squelette non-nucléotide chiralement pur, comportant de 1 à 20 atomes de carbone, où -NHL, Y et Z sont liés par covalence à un atome de carbone de SKEL, L représente un ligand, Y représente -CH₂-, -O-, -S- ou -NH-; et Z représente -O-, -S- ou -NH-.

35. Oligomère selon la revendication 34, dans lequel SKEL comprend un tronc comportant de 1 à 10 atomes de carbone entre Y et Z.

36. Oligomère selon la revendication 35, dans lequel L est choisi parmi -Ps, 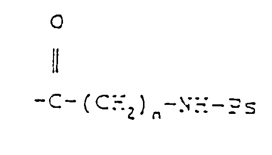 et 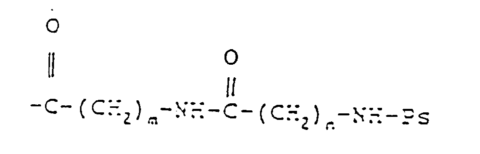 où n et m représentent indépendamment des nombres entiers de 1 à 15, Ps représente un fragment psoralène selon la revendication 6.

37. Oligomère selon la revendication 36 qui comprend un oligomère d'alkyl- ou aryl-phosphonate.

38. Oligomère selon la revendication 36, qui comprend un oligomère de méthylphosphonate.

39. Oligomère selon la revendication 38, qui comprend des motifs monomères nucléosides qui comprennent un fragment 2'-O-méthyl-ribosyle.

40. Oligomère selon la revendication 38, qui comprend de 6 à 31 motifs monomères.

41. Oligomère selon la revendication 40 qui comprend de 1 à 5 motifs monomères non-nucléotides choisis indépendamment.

42. Oligomère selon la revendication 36, qui comprend de 6 à 31 motifs monomères.

43. Oligomère selon la revendication 42, qui comprend de 1 à 5 motifs non-nucléotides choisis indépendamment.

44. Oligomère selon la revendication 33, dans lequel k vaut de 2 à 6.

45. Oligomère selon la revendication 44, dans lequel k est égal à 2.

46. Oligomère selon la revendication 33, dans lequel le motif monomère non-nucléotide comprend : 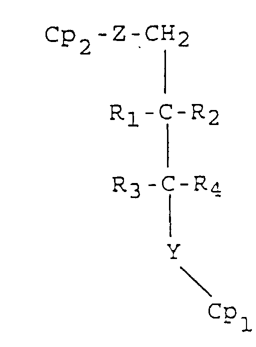 où un des symboles R₁ et R₂ représente un atome d'hydrogène et l'autre représente -NH-L où L représente un bras de liaison conjugué à un fragment psoralène selon la revendication 6; un des symboles R₃ et R₄ représente un atome d'hydrogène et l'autre représente un groupe alkyle inférieur comportant de 1 à 10 atomes de carbone; où -Y-Cp₁ représente un premier groupe de couplage où Y représente -CH₂-, un atome de soufre, un groupe amino ou un atome d'oxygène et Cp₁ est choisi parmi : 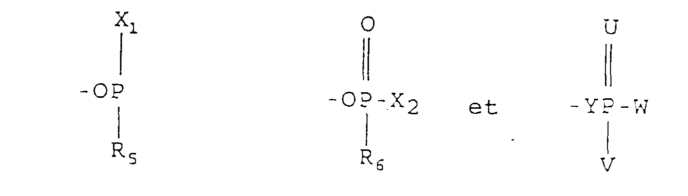 où X₁ représente un atome d'halogène ou un groupe amino substitué; X₂ représente un atome d'halogène, un groupe amino, ou un groupe amino substitué, ou O; R₅ représente un groupe alkyle, un groupe alcoxy substitué ou un groupe aryloxy substitué; et R₆ représente un groupe alkyle, un groupe alcoxy substitué ou un groupe aryloxy substitué ou un atome d'hydrogène quand X₂ représente O; U représente un atome d'oxygène ou de soufre, W représente un groupe alkyle, un groupe aryle, un groupe alcoxy, un groupe alkylthio, un groupe aryloxy, un groupe arylthio, O, S, un groupe amino ou amino substitué, et V représente un groupe alcoxy, un groupe alkylthio, un groupe amino ou amino substitué; et Z-Cp₂ représente un second groupe de couplage bloqué, où Cp₂ représente un groupe de blocage clivable dans des conditions de déblocage pour récupérer le second groupe de couplage -ZH où Z représente -0-, -NH- ou -S-.

47. Oligomère selon la revendication 46, dans lequel L est choisi parmi -Ps 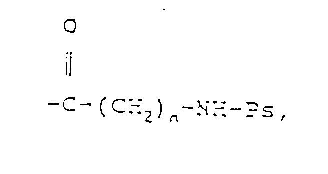 et 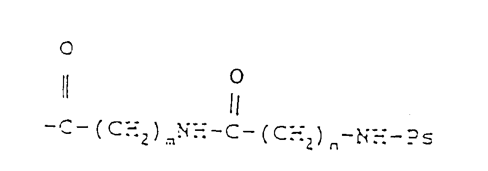 où n et m représentent indépendamment des nombres entiers de 1 à 15 et Ps représente un fragment psoralène tel que défini dans la revendication 6.

48. Oligomère selon la revendication 47, qui comprend un oligomère de méthylphosphonate.

49. Oligomère selon la revendication 48, dans lequel L représente 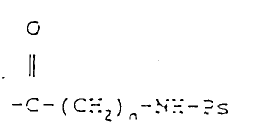 où n est un nombre entier de 1 à 5.

50. Oligomère selon la revendication 49, qui comprend de 6 à 31 motifs monomères.

51. Oligomère selon la revendication 50, qui comprend de 1 à 5 motifs monomères non-nucléotides choisis indépendamment.

52. Oligomère selon la revendication 51, dans lequel lesdits motifs monomères non-nucléotides sont chiralement purs.
